# EUROPEAN PATENT SPECIFICATION

(11) **EP 1 616 013 B1**
(45) Date of publication and mention of the grant of the patent: **27.07.2011**
(21) Application number: 04726719.0
(22) Date of filing: 09.04.2004
(51) Int. Cl.: C12N 15/82

(54) **METHODS AND MEANS FOR INCREASING THE TOLERANCE OF PLANTS TO STRESS CONDITIONS**
VERFAHREN UND MITTEL ZUR ERHÖHUNG DER TOLERANZ VON PFLANZEN GEGENÜBER STRESSBEDINGUNGEN
PROCEDES ET ELEMENTS DESTINES A AUGMENTER LA TOLERANCE DE PLANTES PAR RAPPORT A DES CONDITIONS DE STRESS

(30) Priority: 09.04.2003 EP 03076044; 21.08.2003 US 496688 P
(43) Date of publication of application: 18.01.2006
(73) Proprietor: Bayer BioScience N.V., 9052 Gent (BE)
(72) Inventor: DE BLOCK, Marc, B-9820 Merelbeke (BE)
(86) International application number: PCT/EP2004/003995
(87) International publication number: WO 2004/090140

(56) References cited:
- WO-A-99/53050
- WO-A-00//04173
- WO-A-03/000898
- WO-A-03/008540
- US-A1- 2002 040 490
- PANDA SATCHIDANANDA ET AL: "tej defines a role for poly(ADP-ribosyl)ation in establishing period length of the Arabidopsis circadian oscillator" DEVELOPMENTAL CELL, vol. 3, no. 1, July 2002 (2002-07), pages 51-61, XP009035930 ISSN: 1534-5807
- ALEXANDER BÜRKLE: "Physiology and pathophysiology of poly(ADP-ribosyl)ation" BIOESSAYS, vol. 23, no. 9, pages 795-806, XPXP009120869

### SEQUENCE LISTING

<110> Bayer BioScience N.V.
   De Block, Marc
<120> Methods and means for increasing the tolerance of plants to stress conditions.
<130> BCS 03 2002 WO1
<150> EP03076044.1
   <151> 2003-04-09
<150> US 60/496,688
   <151> 2003-08-21
<160> 25
<170> PatentIn version 3.1
<210> 1
   <211> 548
   <212> PRT
   <213> Arabidopsis thaliana
<400> 1
   Met Glu Asn Arg Glu Asp Leu Asn Ser Ile Leu Pro Tyr Leu Pro Leu
   1               5                   10                  15
   Val Ile Arg Ser Ser Ser Leu Tyr Trp Pro Pro Arg Val Val Glu Ala
               20                  25                  30
   Leu Lys Ala Met Ser Glu Gly Pro Ser His Ser Gln Val Asp Ser Gly
           35                  40                  45
   Glu Val Leu Arg Gln Ala Ile Phe Asp Met Arg Arg Ser Leu Ser Phe
       50                  55                  60
   Ser Thr Leu Glu Pro Ser Ala Ser Asn Gly Tyr Ala Phe Leu Phe Asp
   65                  70                  75                  80
   Glu Leu Ile Asp Glu Lys Glu Ser Lys Arg Trp Phe Asp Glu Ile Ile
                   85                  90                  95
   Pro Ala Leu Ala Ser Leu Leu Leu Gln Phe Pro Ser Leu Leu Glu Val
               100                 105                 110
   His Phe Gln Asn Ala Asp Asn Ile Val Ser Gly Ile Lys Thr Gly Leu
           115                 120                 125
   Arg Leu Leu Asn Ser Gln Gln Ala Gly Ile Val Phe Leu Ser Gln Glu
       130                 135                 140
   Leu Ile Gly Ala Leu Leu Ala Cys Ser Phe Phe Cys Leu Phe Pro Asp
   145                 150                 155                 160
   Asp Asn Arg Gly Ala Lys His Leu Pro Val Ile Asn Phe Asp His Leu
                   165                 170                 175
   Phe Ala Ser Leu Tyr Ile Ser Tyr Ser Gln Ser Gln Glu Ser Lys Ile
               180                 185                 190
   Arg Cys Ile Met His Tyr Phe Glu Arg Phe Cys Ser Cys Val Pro Ile
           195                 200                 205
   Gly Ile Val Ser Phe Glu Arg Lys Ile Thr Ala Ala Pro Asp Ala Asp
       210                 215                 220
   Phe Trp Ser Lys Ser Asp Val Ser Leu Cys Ala Phe Lys Val His Ser
   225                 230                 235                 240
   Phe Gly Leu Ile Glu Asp Gln Pro Asp Asn Ala Leu Glu Val Asp Phe
                   245                 250                 255
   Ala Asn Lys Tyr Leu Gly Gly Gly Ser Leu Ser Arg Gly Cys Val Gln
               260                 265                 270
   Glu Glu Ile Arg Phe Met Ile Asn Pro Glu Leu Ile Ala Gly Met Leu
               275                 280                 285
   Phe Leu Pro Arg Met Asp Asp Asn Glu Ala Ile Glu Ile Val Gly Ala
           290                 295                 300
   Glu Arg Phe Ser Cys Tyr Thr Gly Tyr Ala Ser Ser Phe Arg Phe Ala
   305                 310                 315                 320
   Gly Glu Tyr Ile Asp Lys Lys Ala Met Asp Pro Phe Lys Arg Arg Arg
                   325                 330                 335
   Thr Arg Ile Val Ala Ile Asp Ala Leu Cys Thr Pro Lys Met Arg His
               340                 345                 350
   Phe Lys Asp Ile Cys Leu Leu Arg Glu Ile Asn Lys Ala Leu Cys Gly
           355                 360                 365
   Phe Leu Asn Cys Ser Lys Ala Trp Glu His Gln Asn Ile Phe Met Asp
       370                 375                 380
   Glu Gly Asp Asn Glu Ile Gln Leu Val Arg Asn Gly Arg Asp Ser Gly
   385                 390                 395                 400
   Leu Leu Arg Thr Glu Thr Thr Ala Ser His Arg Thr Pro Leu Asn Asp
                   405                 410                 415
   Val Glu Met Asn Arg Glu Lys Pro Ala Asn Asn Leu Ile Arg Asp Phe
               420                 425                 430
   Tyr Val Glu Gly Val Asp Asn Glu Asp His Glu Asp Asp Gly Val Ala
           435                 440                 445
   Thr Gly Asn Trp Gly Cys Gly Val Phe Gly Gly Asp Pro Glu Leu Lys
       450                 455                 460
   Ala Thr Ile Gln Trp Leu Ala Ala Ser Gln Thr Arg Arg Pro Phe Ile
   465                 470                 475                 480
   Ser Tyr Tyr Thr Phe Gly Val Glu Ala Leu Arg Asn Leu Asp Gln Val
                   485                 490                 495
   Thr Lys Trp Ile Leu Ser His Lys Trp Thr Val Gly Asp Leu Trp Asn
               500                 505                 510
   Met Met Leu Glu Tyr Ser Ala Gln Arg Leu Tyr Lys Gln Thr Ser Val
           515                 520                 525
   Gly Phe Phe Ser Trp Leu Leu Pro Ser Leu Ala Thr Thr Asn Lys Ala
       530                 535                 540
   Ile Gln Pro Pro
   545
<210> 2
   <211> 169
   <212> PRT
   <213> Solanum tuberosum
<400> 2
   Met Glu Asn Arg Glu Asp Val Lys Ser Ile Leu Pro Phe Leu Pro Val
   1               5                   10                  15
   Cys Leu Arg Ser Ser Ser Leu Phe Trp Pro Pro Leu Val Val Glu Ala
               20                  25                  30
   Leu Lys Ala Leu Ser Glu Gly Pro His Tyr Ser Asn Val Asn Ser Gly
           35                  40                  45
   Gln Val Leu Phe Leu Ala Ile Ser Asp Ile Arg Asn Ser Leu Ser Leu
       50                  55                  60
   Pro Asp Ser Ser Ile Ser Ser Ser Ala Ser Asp Gly Phe Ser Leu Leu
   65                  70                  75                  80
   Phe Asp Asp Leu Ile Pro Arg Asp Glu Ala Val Lys Trp Phe Lys Glu
                   85                  90                  95
   Val Val Pro Lys Met Ala Asp Leu Leu Leu Arg Leu Pro Ser Leu Leu
               100                 105                 110
   Glu Ala His Tyr Glu Lys Ala Asp Gly Gly Ile Val Lys Gly Val Asn
           115                 120                 125
   Thr Gly Leu Arg Leu Leu Glu Ser Gln Gln Pro Gly Ile Val Phe Leu
       130                 135                 140
   Ser Gln Glu Leu Val Gly Ala Leu Leu Ala Cys Ser Phe Phe Cys Tyr
   145                 150                 155                 160
   Ser Leu Pro Met Ile Glu Val Ser Val
                   165
<210> 3
   <211> 1647
   <212> DNA
   <213> Arabidopsis thaliana
<400> 3
   atggagaatc gcgaagatct taactcaatt cttccgtacc ttccacttgt aattcgttcg     60
   tcgtcgctgt attggccgcc gcgtgtggtg gaggcgttaa aggcaatgtc tgaaggacca    120
   tctcacagcc aagttgactc aggagaggtt ctacggcaag ctattttcga tatgagacga    180
   tccttatctt tctctactct cgagccatct gcttctaatg gctacgcatt tctctttgac    240
   gaattgattg atgagaaaga atcaaagaga tggttcgatg agattatccc agcattggcg    300
   agcttacttc tacagtttcc atctctgtta gaagtgcatt tccaaaatgc tgataatatt    360
   gttagtggaa tcaaaaccgg tcttcgtttg ttaaattccc aacaagctgg cattgttttc    420
   ctcagccagg agttgattgg agctcttctt gcatgctctt tcttttgttt gtttccggat    480
   gataatagag gtgcaaaaca ccttccagtc atcaactttg atcatttgtt tgcaagcctt    540
   tatataagtt atagtcaaag tcaagaaagc aagataagat gtattatgca ttactttgaa    600
   aggttttgct cctgcgtgcc tattggtatt gtttcatttg aacgcaagat taccgctgct    660
   cctgatgctg atttctggag caagtctgac gtttctcttt gtgcatttaa ggttcactct    720
   tttgggttaa ttgaagatca acctgacaat gctctcgaag tggactttgc aaacaagtat    780
   ctcggaggtg gttccctaag tagagggtgc gtgcaggaag agatacgctt catgattaac    840
   cctgaattaa tcgctggcat gcttttcttg cctcggatgg atgacaatga agctatagaa    900
   atagttggtg cggaaagatt ttcatgttac acagggtatg catcttcgtt tcggtttgct    960
   ggtgagtaca ttgacaaaaa ggcaatggat cctttcaaaa ggcgaagaac cagaattgtt   1020
   gcaattgatg cattatgtac accgaagatg agacacttta aagatatatg tcttttaagg   1080
   gaaattaata aggcactatg tggcttttta aattgtagca aggcttggga gcaccagaat   1140
   atattcatgg atgaaggaga taatgaaatt cagcttgtcc gaaacggcag agattctggt   1200
   cttctgcgta cagaaactac tgcgtcacac cgaactccac taaatgatgt tgagatgaat   1260
   agagaaaagc ctgctaacaa tcttatcaga gatttttatg tggaaggagt tgataacgag   1320
   gatcatgaag atgatggtgt cgcgacaggg aattggggat gtggtgtttt tggaggagac   1380
   ccagagctaa aggctacgat acaatggctt gctgcttccc agactcgaag accatttata   1440
   tcatattaca cctttggagt agaggcactc cgaaacctag atcaggtgac gaagtggatt   1500
   ctttcccata aatggactgt tggagatctg tggaacatga tgttagaata ttctgctcaa   1560
   aggctctaca agcaaaccag tgttggcttc ttttcttggc tacttccatc tctagctacc   1620
   accaacaaag ctatccagcc gccttga                                        1647
<210> 4
   <211> 598
   <212> DNA
   <213> Solanum tuberosum
<400> 4
   gcaatggaga atagagaaga cgtgaagtca atccttccct ttttgccggt gtgtctccga     60
   tcatcttctc ttttctggcc gccgctagtt gttgaagcac tgaaagccct ctctgaaggc    120
   cctcattaca gcaatgttaa ctccggccaa gtcctcttcc tcgcaatctc cgacattcgg    180
   aattcccttt cactacctga ttcttcaatt tcctcttctg cttcagacgg attttctctc    240
   ttatttgatg atttaattcc tagggatgaa gctgttaaat ggttcaaaga agtggtgccg    300
   aaaatggcgg atttgctatt gcggttgcct tccttattgg aggctcacta tgagaaggct    360
   gatggtggaa ttgttaaagg agtcaacact ggtcttcgct tattggaatc acaacagcct    420
   ggcattgttt tcctcagtca ggaattagtc ggtgctcttc ttgcatgttc cttcttttgc    480
   tattccctac caatgataga ggtatctgta tgatcagtat gacgagaaat ttgaaaataa    540
   attgaagtgc attcttcact attttgagag gattggctca ttgatacctg cgggctac      598
<210> 5
   <211> 37
   <212> DNA
   <213> Artificial Sequence
<220>
   <223> oligonucleotide primer ParGAt1
   <400> 5
   ggatcccctg caggacaaaa aggcaatgga tcctttc 37
<210> 6
   <211> 39
   <212> DNA
   <213> Artificial Sequence
<220>
   <223> oligonucleotide primer ParGAt2
   <400> 6
   gcacgaattc gcggccgcgg tgctcccaag ccttgctac 39
<210> 7
   <211> 39
   <212> DNA
   <213> Artificial Sequence
<220>
   <223> oligonucleotide primer ParGSt1
   <400> 7
   ggatcccctg caggctcact atgagaaggc tgatggtgg 39
<210> 8
   <211> 43
   <212> DNA
   <213> Artificial Sequence
<220>
   <223> oligonucleotide primer ParGSt2
   <400> 8
   gcacgaattc gcggccgcgt catactgatc atacagatac ctc 43
<210> 9
   <211> 13466
   <212> DNA
   <213> Artificial Sequence
<220>
   <223> nucleotide sequence of pTVE428
   <220>
   <221> misc_feature
   <222> (198)..(222)
   <223> Right T-DNA border
<220>
   <221> misc_feature
   <222> (983)..(273)
   <223> 3' ocs (3' untranslated end of octopine synthase gene)
<220>
   <221> misc_feature
   <222> (995)..(1155)
   <223> part of poly (ADP-ribose) glycohydrolase
<220>
   <221> misc_feature
   <222> (1929)..(1188)
   <223> intron 2 from the Pdk gene of Flaveria
<220>
   <221> misc_feature
   <222> (2122)..(1962)
   <223> part of poly (ADP-ribose) glycohydrolase
<220>
   <221> misc_feature
   <222> (3476)..(2131)
   <223> 35S promoter region from Cauliflower Mosaic Virus
<220>
   <221> misc_feature
   <222> (3948)..(3737)
   <223> 3' untranslated end of gene 7 from Agrobacterium tumefaciens
<220>
   <221> misc_feature
   <222> (4521)..(3970)
   <223> bar coding region
<220>
   <221> misc_feature
   <222> (6247)..(4522)
   <223> PSSuAra promoter region
<220>
   <221> misc_feature
   <222> (6415)..(6439)
   <223> left border region of T-DNA of Agrobacterium tumefaciens
<400> 9
   agattcgaag ctcggtcccg tgggtgttct gtcgtctcgt tgtacaacga aatccattcc     60
   cattccgcgc tcaagatggc ttcccctcgg cagttcatca gggctaaatc aatctagccg    120
   acttgtccgg tgaaatgggc tgcactccaa cagaaacaat caaacaaaca tacacagcga    180
   cttattcaca cgcgacaaat tacaacggta tatatcctgc cagtactcgg ccgtcgaccg    240
   cggtaccccg gaattaagct tgcatgcctg caggtcctgc tgagcctcga catgttgtcg    300
   caaaattcgc cctggacccg cccaacgatt tgtcgtcact gtcaaggttt gacctgcact    360
   tcatttgggg cccacataca ccaaaaaaat gctgcataat tctcggggca gcaagtcggt    420
   tacccggccg ccgtgctgga ccgggttgaa tggtgcccgt aactttcggt agagcggacg    480
   gccaatactc aacttcaagg aatctcaccc atgcgcgccg gcggggaacc ggagttccct    540
   tcagtgaacg ttattagttc gccgctcggt gtgtcgtaga tactagcccc tggggccttt    600
   tgaaatttga ataagattta tgtaatcagt cttttaggtt tgaccggttc tgccgctttt    660
   tttaaaattg gatttgtaat aataaaacgc aattgtttgt tattgtggcg ctctatcata    720
   gatgtcgcta taaacctatt cagcacaata tattgttttc attttaatat tgtacatata    780
   agtagtaggg tacaatcagt aaattgaacg gagaatatta ttcataaaaa tacgatagta    840
   acgggtgata tattcattag aatgaaccga aaccggcggt aaggatctga gctacacatg    900
   ctcaggtttt ttacaacgtg cacaacagaa ttgaaagcaa atatcatgcg atcataggcg    960
   tctcgcatat ctcattaaag caggactcta gagacaaaaa ggcaatggat cctttcaaaa   1020
   ggcgaagaac cagaattgtt gcaattgatg cattatgtac accgaagatg agacacttta   1080
   aagatatatg tcttttaagg gaaattaata aggcactatg tggcttttta aattgtagca   1140
   aggcttggga gcaccatcga tttcgaaccc agcttcccaa ctgtaatcaa tccaaatgta   1200
   agatcaatga taacacaatg acatgatcta tcatgttacc ttgtttattc atgttcgact   1260
   aattcattta attaatagtc aatccattta gaagttaata aaactacaag tattatttag   1320
   aaattaataa gaatgttgat tgaaaataat actatataaa atgatagatc ttgcgctttg   1380
   ttatattagc attagattat gttttgttac attagattac tgtttctatt agtttgatat   1440
   tatttgttac tttagcttgt tatttaatat tttgtttatt gataaattac aagcagattg 1500
   gaatttctaa caaaatattt attaactttt aaactaaaat atttagtaat ggtatagata 1560
   tttaattata taataaacta ttaatcataa aaaaatatta ttttaattta tttattctta 1620
   tttttactat agtattttat cattgatatt taattcatca aaccagctag aattactatt 1680
   atgattaaaa caaatattaa tgctagtata tcatcttaca tgttcgatca aattcattaa 1740
   aaataatata cttactctca acttttatct tcttcgtctt acacatcact tgtcatattt 1800
   ttttacatta ctatgttgtt tatgtaaaca atatatttat aaattatttt ttcacaatta 1860
   taacaactat attattataa tcatactaat taacatcact taactatttt atactaaaag 1920
   gaaaaaagaa aataattatt tccttaccaa gctggggtac cggtgctccc aagccttgct 1980
   acaatttaaa aagccacata gtgccttatt aatttccctt aaaagacata tatctttaaa 2040
   gtgtctcatc ttcggtgtac ataatgcatc aattgcaaca attctggttc ttcgcctttt 2100
   gaaaggatcc attgcctttt tgtcctcgag cgtgtcctct ccaaatgaaa tgaacttcct 2160
   tatatagagg aagggtcttg cgaaggatag tgggattgtg cgtcatccct tacgtcagtg 2220
   gagatgtcac atcaatccac ttgctttgaa gacgtggttg gaacgtcttc tttttccacg 2280
   atgctcctcg tgggtggggg tccatctttg ggaccactgt cggcagagag atcttgaatg 2340
   atagcctttc ctttatcgca atgatggcat ttgtaggagc caccttcctt ttctactgtc 2400
   ctttcgatga agtgacagat agctgggcaa tggaatccga ggaggtttcc cgaaattatc 2460
   ctttgttgaa aagtctcaat agccctttgg tcttctgaga ctgtatcttt gacatttttg 2520
   gagtagacca gagtgtcgtg ctccaccatg ttgacgaaga ttttcttctt gtcattgagt 2580
   cgtaaaagac tctgtatgaa ctgttcgcca gtcttcacgg cgagttctgt tagatcctcg 2640
   atttgaatct tagactccat gcatggcctt agattcagta ggaactacct ttttagagac 2700
   tccaatctct attacttgcc ttggtttatg aagcaagcct tgaatcgtcc atactggaat 2760
   agtacttctg atcttgagaa atatgtcttt ctctgtgttc ttgatgcaat tagtcctgaa 2820
   tcttttgact gcatctttaa ccttcttggg aaggtatttg atctcctgga gattgttact 2880
   cgggtagatc gtcttgatga gacctgctgc gtaggcctct ctaaccatct gtgggtcagc 2940
   attctttctg aaattgaaga ggctaacctt ctcattatca gtggtgaaca tagtgtcgtc 3000
   accttcacct tcgaacttcc ttcctagatc gtaaagatag aggaaatcgt ccattgtaat 3060
   ctccggggca aaggagatct cttttggggc tggatcactg ctgggccttt tggttcctag 3120
   cgtgagccag tgggcttttt gctttggtgg gcttgttagg gccttagcaa agctcttggg 3180
   cttgagttga gcttctcctt tggggatgaa gttcaacctg tctgtttgct gacttgttgt 3240
   gtacgcgtca gctgctgctc ttgcctctgt aatagtggca aatttcttgt gtgcaactcc 3300
   gggaacgccg tttgttgccg cctttgtaca accccagtca tcgtatatac cggcatgtgg 3360
   accgttatac acaacgtagt agttgatatg agggtgttga atacccgatt ctgctctgag 3420
   aggagcaact gtgctgttaa gctcagattt ttgtgggatt ggaattaatt cgtcgagcgg 3480
   ccgctcgacg agcgcgccga tatcgcgatc gcccgggccg gccatttaaa tgaattcgag 3540
   ctcggtaccc aaacgcggcc gcaagctata acttcgtata gcatacatta tacgaagtta 3600
   ttcgactcta gaggatccca attcccatgc atggagtcaa agattcaaat agaggacact 3660
   tctcgaactc ggccgtcgaa ctcggccgtc gagtacatgg tcgataagaa aaggcaattt 3720
   gtagatgtta attcccatct tgaaagaaat atagtttaaa tatttattga taaaataaca 3780
   agtcaggtat tatagtccaa gcaaaaacat aaatttattg atgcaagttt aaattcagaa 3840
   atatttcaat aactgattat atcagctggt acattgccgt agatgaaaga ctgagtgcga 3900
   tattatgtgt aatacataaa ttgatgatat agctagctta gctcatcggg ggatcctaga 3960
   cgcgtgagat cagatctcgg tgacgggcag gaccggacgg ggcggtaccg gcaggctgaa 4020
   gtccagctgc cagaaaccca cgtcatgcca gttcccgtgc ttgaagccgg ccgcccgcag 4080
   catgccgcgg ggggcatatc cgagcgcctc gtgcatgcgc acgctcgggt cgttgggcag 4140
   cccgatgaca gcgaccacgc tcttgaagcc ctgtgcctcc agggacttca gcaggtgggt 4200
   gtagagcgtg gagcccagtc ccgtccgctg gtggcggggg gagacgtaca cggtcgactc 4260
   ggccgtccag tcgtaggcgt tgcgtgcctt ccagggcccc gcgtaggcga tgccggcgac 4320
   ctcgccgtcc acctcggcga cgagccaggg atagcgctcc cgcagacgga cgaggtcgtc 4380
   cgtccactcc tgcggttcct gcggctcggt acggaagttg accgtgcttg tctcgatgta 4440
   gtggttgacg atggtgcaga ccgccggcat gtccgcctcg gtggcacggc ggatgtcggc 4500
   cgggcgtcgt tctgggtcca ttgttcttct ttactctttg tgtgactgag gtttggtcta 4560
   gtgctttggt catctatata taatgataac aacaatgaga acaagctttg gagtgatcgg 4620
   agggtctagg atacatgaga ttcaagtgga ctaggatcta caccgttgga ttttgagtgt 4680
   ggatatgtgt gaggttaatt ttacttggta acggccacaa aggcctaagg agaggtgttg 4740
   agacccttat cggcttgaac cgctggaata atgccacgtg gaagataatt ccatgaatct 4800
   tatcgttatc tatgagtgaa attgtgtgat ggtggagtgg tgcttgctca ttttacttgc 4860
   ctggtggact tggccctttc cttatgggga atttatattt tacttactat agagctttca 4920
   tacctttttt ttaccttgga tttagttaat atataatggt atgattcatg aataaaaatg 4980
   ggaaattttt gaatttgtac tgctaaatgc ataagattag gtgaaactgt ggaatatata 5040
   tttttttcat ttaaaagcaa aatttgcctt ttactagaat tataaatata gaaaaatata 5100
   taacattcaa ataaaaatga aaataagaac tttcaaaaaa cagaactatg tttaatgtgt 5160
   aaagattagt cgcacatcaa gtcatctgtt acaatatgtt acaacaagtc ataagcccaa 5220
   caaagttagc acgtctaaat aaactaaaga gtccacgaaa atattacaaa tcataagccc 5280
   aacaaagtta ttgatcaaaa aaaaaaaacg cccaacaaag ctaaacaaag tccaaaaaaa 5340
   acttctcaag tctccatctt cctttatgaa cattgaaaac tatacacaaa acaagtcaga 5400
   taaatctctt tctgggcctg tcttcccaac ctcctacatc acttccctat cggattgaat 5460
   gttttacttg taccttttcc gttgcaatga tattgatagt atgtttgtga aaactaatag 5520
   ggttaacaat cgaagtcatg gaatatggat ttggtccaag attttccgag agctttctag 5580
   tagaaagccc atcaccagaa atttactagt aaaataaatc accaattagg tttcttatta 5640
   tgtgccaaat tcaatataat tatagaggat atttcaaatg aaaacgtatg aatgttatta 5700
   gtaaatggtc aggtaagaca ttaaaaaaat cctacgtcag atattcaact ttaaaaattc 5760
   gatcagtgtg gaattgtaca aaaatttggg atctactata tatatataat gctttacaac 5820
   acttggattt ttttttggag gctggaattt ttaatctaca tatttgtttt ggccatgcac 5880
   caactcattg tttagtgtaa tactttgatt ttgtcaaata tatgtgttcg tgtatatttg 5940
   tataagaatt tctttgacca tatacacaca cacatatata tatatatata tatattatat 6000
   atcatgcact tttaattgaa aaaataatat atatatatat agtgcatttt ttctaacaac 6060
   catatatgtt gcgattgatc tgcaaaaata ctgctagagt aatgaaaaat ataatctatt 6120
   gctgaaatta tctcagatgt taagattttc ttaaagtaaa ttctttcaaa ttttagctaa 6180
   aagtcttgta ataactaaag aataatacac aatctcgacc acggaaaaaa aacacataat 6240
   aaatttgaat ttcgaccgcg gtacccggaa ttgggttata attacctcag gtcgaggaat 6300
   taattcggta cgtacctaat aacttcgtat agcatacatt atacgaagtt atatggatct 6360
   cgaggcatta cggcattacg gcactcgcga gggtcccaat tcgagcatgg agccatttac 6420
   aattgaatat atcctgccgc cgctgccgct ttgcacccgg tggagcttgc atgttggttt 6480
   ctacgcagaa ctgagccggt taggcagata atttccattg agaactgagc catgtgcacc 6540
   ttccccccaa cacggtgagc gacggggcaa cggagtgatc cacatgggac ttttaaacat 6600
   catccgtcgg atggcgttgc gagagaagca gtcgatccgt gagatcagcc gacgcaccgg 6660
   gcaggcgcgc aacacgatcg caaagtattt gaacgcaggt acaatcgagc cgacgttcac 6720
   ggtaccggaa cgaccaagca agctagctta gtaaagccct cgctagattt taatgcggat 6780
   gttgcgatta cttcgccaac tattgcgata acaagaaaaa gccagccttt catgatatat 6840
   ctcccaattt gtgtagggct tattatgcac gcttaaaaat aataaaagca gacttgacct 6900
   gatagtttgg ctgtgagcaa ttatgtgctt agtgcatcta acgcttgagt taagccgcgc 6960
   cgcgaagcgg cgtcggcttg aacgaattgt tagacattat ttgccgacta ccttggtgat 7020
   ctcgcctttc acgtagtgga caaattcttc caactgatct gcgcgcgagg ccaagcgatc 7080
   ttcttcttgt ccaagataag cctgtctagc ttcaagtatg acgggctgat actgggccgg 7140
   caggcgctcc attgcccagt cggcagcgac atccttcggc gcgattttgc cggttactgc 7200
   gctgtaccaa atgcgggaca acgtaagcac tacatttcgc tcatcgccag cccagtcggg 7260
   cggcgagttc catagcgtta aggtttcatt tagcgcctca aatagatcct gttcaggaac 7320
   cggatcaaag agttcctccg ccgctggacc taccaaggca acgctatgtt ctcttgcttt 7380
   tgtcagcaag atagccagat caatgtcgat cgtggctggc tcgaagatac ctgcaagaat 7440
   gtcattgcgc tgccattctc caaattgcag ttcgcgctta gctggataac gccacggaat 7500
   gatgtcgtcg tgcacaacaa tggtgacttc tacagcgcgg agaatctcgc tctctccagg 7560
   ggaagccgaa gtttccaaaa ggtcgttgat caaagctcgc cgcgttgttt catcaagcct 7620
   tacggtcacc gtaaccagca aatcaatatc actgtgtggc ttcaggccgc catccactgc 7680
   ggagccgtac aaatgtacgg ccagcaacgt cggttcgaga tggcgctcga tgacgccaac 7740
   tacctctgat agttgagtcg atacttcggc gatcaccgct tccctcatga tgtttaactt 7800
   tgttttaggg cgactgccct gctgcgtaac atcgttgctg ctccataaca tcaaacatcg 7860
   acccacggcg taacgcgctt gctgcttgga tgcccgaggc atagactgta ccccaaaaaa 7920
   acagtcataa caagccatga aaaccgccac tgcgccgtta ccaccgctgc gttcggtcaa 7980
   ggttctggac cagttgcgtg agcgcatacg ctacttgcat tacagcttac gaaccgaaca 8040
   ggcttatgtc cactgggttc gtgccttcat ccgtttccac ggtgtgcgtc acccggcaac 8100
   cttgggcagc agcgaagtcg aggcatttct gtcctggctg gcgaacgagc gcaaggtttc 8160
   ggtctccacg catcgtcagg cattggcggc cttgctgttc ttctacggca agtgctgtgc 8220
   acggatctgc cctggcttca ggagatcgga agacctcggc cgtccgggcg cttgccggtg 8280
   gtgctgaccc cggatgaagt ctctagagct ctagagggtt cgcatcctcg gttttctgga 8340
   aggcgagcat cgtttgttcg cccagcttct gtatggaacg ggcatgcgga tcagtgaggg 8400
   tttgcaactg cgggtcaagg atctggattt cgatcacggc acgatcatcg tgcgggaggg 8460
   caagggctcc aaggatcggg ccttgatgtt acccgagagc ttggcaccca gcctgcgcga 8520
   gcagggatcg atccaacccc tccgctgcta tagtgcagtc ggcttctgac gttcagtgca 8580
   gccgtcttct gaaaacgaca tgtcgcacaa gtcctaagtt acgcgacagg ctgccgccct 8640
   gcccttttcc tggcgttttc ttgtcgcgtg ttttagtcgc ataaagtaga atacttgcga 8700
   ctagaaccgg agacattacg ccatgaacaa gagcgccgcc gctggcctgc tgggctatgc 8760
   ccgcgtcagc accgacgacc aggacttgac caaccaacgg gccgaactgc acgcggccgg 8820
   ctgcaccaag ctgttttccg agaagatcac cggcaccagg cgcgaccgcc cggagctggc 8880
   caggatgctt gaccacctac gccctggcga cgttgtgaca gtgaccaggc tagaccgcct 8940
   ggcccgcagc acccgcgacc tactggacat tgccgagcgc atccaggagg ccggcgcggg 9000
   cctgcgtagc ctggcagagc cgtgggccga caccaccacg ccggccggcc gcatggtgtt 9060
   gaccgtgttc gccggcattg ccgagttcga gcgttcccta atcatcgacc gcacccggag 9120
   cgggcgcgag gccgccaagg cccgaggcgt gaagtttggc ccccgcccta ccctcacccc 9180
   ggcacagatc gcgcacgccc gcgagctgat cgaccaggaa ggccgcaccg tgaaagaggc 9240
   ggctgcactg cttggcgtgc atcgctcgac cctgtaccgc gcacttgagc gcagcgagga 9300
   agtgacgccc accgaggcca ggcggcgcgg tgccttccgt gaggacgcat tgaccgaggc 9360
   cgacgccctg gcggccgccg agaatgaacg ccaagaggaa caagcatgaa accgcaccag 9420
   gacggccagg acgaaccgtt tttcattacc gaagagatcg aggcggagat gatcgcggcc 9480
   gggtacgtgt tcgagccgcc cgcgcacgtc tcaaccgtgc ggctgcatga aatcctggcc 9540
   ggtttgtctg atgccaagct ggcggcctgg ccggccagct tggccgctga agaaaccgag 9600
   cgccgccgtc taaaaaggtg atgtgtattt gagtaaaaca gcttgcgtca tgcggtcgct 9660
   gcgtatatga tgcgatgagt aaataaacaa atacgcaagg ggaacgcatg aaggttatcg 9720
   ctgtacttaa ccagaaaggc gggtcaggca agacgaccat cgcaacccat ctagcccgcg 9780
   ccctgcaact cgccggggcc gatgttctgt tagtcgattc cgatccccag ggcagtgccc 9840
   gcgattgggc ggccgtgcgg gaagatcaac cgctaaccgt tgtcggcatc gaccgcccga 9900
   cgattgaccg cgacgtgaag gccatcggcc ggcgcgactt cgtagtgatc gacggagcgc 9960
   cccaggcggc ggacttggct gtgtccgcga tcaaggcagc cgacttcgtg ctgattccgg 10020
   tgcagccaag cccttacgac atatgggcca ccgccgacct ggtggagctg gttaagcagc 10080
   gcattgaggt cacggatgga aggctacaag cggcctttgt cgtgtcgcgg gcgatcaaag 10140
   gcacgcgcat cggcggtgag gttgccgagg cgctggccgg gtacgagctg cccattcttg 10200
   agtcccgtat cacgcagcgc gtgagctacc caggcactgc cgccgccggc acaaccgttc 10260
   ttgaatcaga acccgagggc gacgctgccc gcgaggtcca ggcgctggcc gctgaaatta 10320
   aatcaaaact catttgagtt aatgaggtaa agagaaaatg agcaaaagca caaacacgct 10380
   aagtgccggc cgtccgagcg cacgcagcag caaggctgca acgttggcca gcctggcaga 10440
   cacgccagcc atgaagcggg tcaactttca gttgccggcg gaggatcaca ccaagctgaa 10500
   gatgtacgcg gtacgccaag gcaagaccat taccgagctg ctatctgaat acatcgcgca 10560
   gctaccagag taaatgagca aatgaataaa tgagtagatg aattttagcg gctaaaggag 10620
   gcggcatgga aaatcaagaa caaccaggca ccgacgccgt ggaatgcccc atgtgtggag 10680
   gaacgggcgg ttggccaggc gtaagcggct gggttgtctg ccggccctgc aatggcactg 10740
   gaacccccaa gcccgaggaa tcggcgtgac ggtcgcaaac catccggccc ggtacaaatc 10800
   ggcgcggcgc tgggtgatga cctggtggag aagttgaagg ccgcgcaggc cgcccagcgg 10860
   caacgcatcg aggcagaagc acgccccggt gaatcgtggc aagcggccgc tgatcgaatc 10920
   cgcaaagaat cccggcaacc gccggcagcc ggtgcgccgt cgattaggaa gccgcccaag 10980
   ggcgacgagc aaccagattt tttcgttccg atgctctatg acgtgggcac ccgcgatagt 11040
   cgcagcatca tggacgtggc cgttttccgt ctgtcgaagc gtgaccgacg agctggcgag 11100
   gtgatccgct acgagcttcc agacgggcac gtagaggttt ccgcagggcc ggccggcatg 11160
   gccagtgtgt gggattacga cctggtactg atggcggttt cccatctaac cgaatccatg 11220
   aaccgatacc gggaagggaa gggagacaag cccggccgcg tgttccgtcc acacgttgcg 11280
   gacgtactca agttctgccg gcgagccgat ggcggaaagc agaaagacga cctggtagaa 11340
   acctgcattc ggttaaacac cacgcacgtt gccatgcagc gtacgaagaa ggccaagaac 11400
   ggccgcctgg tgacggtatc cgagggtgaa gccttgatta gccgctacaa gatcgtaaag 11460
   agcgaaaccg ggcggccgga gtacatcgag atcgagctag ctgattggat gtaccgcgag 11520
   atcacagaag gcaagaaccc ggacgtgctg acggttcacc ccgattactt tttgatcgat 11580
   cccggcatcg gccgttttct ctaccgcctg gcacgccgcg ccgcaggcaa ggcagaagcc 11640
   agatggttgt tcaagacgat ctacgaacgc agtggcagcg ccggagagtt caagaagttc 11700
   tgtttcaccg tgcgcaagct gatcgggtca aatgacctgc cggagtacga tttgaaggag 11760
   gaggcggggc aggctggccc gatcctagtc atgcgctacc gcaacctgat cgagggcgaa 11820
   gcatccgccg gttcctaatg tacggagcag atgctagggc aaattgccct agcaggggaa 11880
   aaaggtcgaa aaggtctctt tcctgtggat agcacgtaca ttgggaaccc aaagccgtac 11940
   attgggaacc ggaacccgta cattgggaac ccaaagccgt acattgggaa ccggtcacac 12000
   atgtaagtga ctgatataaa agagaaaaaa ggcgattttt ccgcctaaaa ctctttaaaa 12060
   cttattaaaa ctcttaaaac ccgcctggcc tgtgcataac tgtctggcca gcgcacagcc 12120
   gaagagctgc aaaaagcgcc tacccttcgg tcgctgcgct ccctacgccc cgccgcttcg 12180
   cgtcggccta tcgcggccgc tggccgctca aaaatggctg gcctacggcc aggcaatcta 12240
   ccagggcgcg gacaagccgc gccgtcgcca ctcgaccgcc ggcgcccaca tcaaggcacc 12300
   ctgcctcgcg cgtttcggtg atgacggtga aaacctctga cacatgcagc tcccggagac 12360
   ggtcacagct tgtctgtaag cggatgccgg gagcagacaa gcccgtcagg gcgcgtcagc 12420
   gggtgttggc gggtgtcggg gcgcagccat gacccagtca cgtagcgata gcggagtgta  12480
   tactggctta actatgcggc atcagagcag attgtactga gagtgcacca tatgcggtgt  12540
   gaaataccgc acagatgcgt aaggagaaaa taccgcatca ggcgctcttc cgcttcctcg  12600
   ctcactgact cgctgcgctc ggtcgttcgg ctgcggcgag cggtatcagc tcactcaaag  12660
   gcggtaatac ggttatccac agaatcaggg gataacgcag gaaagaacat gtgagcaaaa  12720
   ggccagcaaa aggccaggaa ccgtaaaaag gccgcgttgc tggcgttttt ccataggctc  12780
   cgcccccctg acgagcatca caaaaatcga cgctcaagtc agaggtggcg aaacccgaca  12840
   ggactataaa gataccaggc gtttccccct ggaagctccc tcgtgcgctc tcctgttccg  12900
   accctgccgc ttaccggata cctgtccgcc tttctccctt cgggaagcgt ggcgctttct  12960
   catagctcac gctgtaggta tctcagttcg gtgtaggtcg ttcgctccaa gctgggctgt  13020
   gtgcacgaac cccccgttca gcccgaccgc tgcgccttat ccggtaacta tcgtcttgag  13080
   tccaacccgg taagacacga cttatcgcca ctggcagcag ccactggtaa caggattagc  13140
   agagcgaggt atgtaggcgg tgctacagag ttcttgaagt ggtggcctaa ctacggctac  13200
   actagaagga cagtatttgg tatctgcgct ctgctgaagc cagttacctt cggaaaaaga  13260
   gttggtagct cttgatccgg caaacaaacc accgctggta gcggtggttt ttttgtttgc  13320
   aagcagcaga ttacgcgcag aaaaaaagga tctcaagaag atccggaaaa cgcaagcgca  13380
   aagagaaagc aggtagcttg cagtgggctt acatggcgat agctagactg ggcggtttta  13440
   tggacagcaa gcgaaccgga attgcc                                        13466
<210> 10
   <211> 31
   <212> PRT
   <213> Artificial Sequence
<220>
   <223> Consensus sequence 1 of PARG protein
   <220>
   <221> MISC_FEATURE
   <222> (2).. (2)
   <223> X represents any amino acid
<220>
   <221> MISC_FEATURE
   <222> (8)..(10)
   <223> X represents any amino acid
<220>
   <221> MISC_FEATURE
   <222> (14)..(17)
   <223> X represents any amino acid
<220>
   <221> MISC FEATURE
   <222> (19)..(19)
   <223> X represents any amino acid
<220>
   <221> MISC FEATURE
   <222> (27)..(29)
   <223> X represents any amino acid
<400> 10
   Leu Xaa Val Asp Phe Ala Asn Xaa Xaa Xaa Gly Gly Gly Xaa Xaa Xaa
   1               5                   10                  15
   Xaa Gly Xaa Val Gln Glu Glu Ile Arg Phe Xaa Xaa Xaa Pro Glu
               20                  25                  30
<210> 11
   <211> 20
   <212> PRT
   <213> Artificial Sequence
<220>
   <223> Consensus sequence 2 for PARG protein
   <220>
   <221> MISC_FEATURE
   <222> (3)..(3)
   <223> X represents any amino acid
<220>
   <221> MISC_FEATURE
   <222> (10)..(10)
   <223> X represents any amino acid
<220>
   <221> MISC_FEATURE
   <222> (13)..(14)
   <223> X represents any amino acid
<220>
   <221> MISC_FEATURE
   <222> (17)..(19)
   <223> X represents any amino acid
<400> 11
   Thr Gly Xaa Trp Gly Cys Gly Ala Phe Xaa Gly Asp Xaa Xaa Leu Lys
   1               5                   10                  15
   Xaa Xaa Xaa Gln
               20
<210> 12
   <211> 13
   <212> PRT
   <213> Artificial Sequence
<220>
   <223> Consensus sequence 3 for PARG protein
   <220>
   <221> MISC_FEATURE
   <222> (2)..(4)
   <223> X represents any amino acid
<220>
   <221> MISC_FEATURE
   <222> (6)..(9)
   <223> X represents any amino acid
<400> 12
   Asp Xaa Xaa Xaa Arg Xaa Xaa Xaa Xaa Ala Ile Asp Ala
   1 5 10
<210> 13
   <211> 10
   <212> PRT
   <213> Artificial Sequence
<220>
   <223> Consensus sequence 4 for PARG protein
   <220>
   <221> MISC_FEATURE
   <222> (3)..(4)
   <223> X represents any amino acid
<220>
   <221> MISC_FEATURE
   <222> (7)..(8)
   <223> X represents any amino acid
<400> 13
   Arg Glu Xaa Xaa Lys Ala Xaa Xaa Gly Phe
   1 5 10
<210> 14
   <211> 11
   <212> PRT
   <213> Artificial Sequence
<220>
   <223> conserved PARG region
   <220>
   <221> MISC_FEATURE
   <222> (2)..(5)
   <223> X represents any amino acid
<220>
   <221> MISC_FEATURE
   <222> (7)..(7)
   <223> X represents any amino acid
<400> 14
   Gly Xaa Xaa Xaa Xaa Ser Xaa Tyr Thr Gly Tyr
   1 5 10
<210> 15
   <211> 1530
   <212> DNA
   <213> Oryza sativa
<220>
   <221> CDS
   <222> (1)..(1530)
   <223>
<400> 15
   atg gag gcg cgc ggc gac ctg cgc tcg atc ctg ccc tac ctc ccc gtc        48
   Met Glu Ala Arg Gly Asp Leu Arg Ser Ile Leu Pro Tyr Leu Pro Val
   1               5                   10                  15
   gtg ctc cgc ggc ggc gcg ctc ttc tgg ccg ccg gcg gcg cag gag gcg        96
   Val Leu Arg Gly Gly Ala Leu Phe Trp Pro Pro Ala Ala Gln Glu Ala
               20                  25                  30
   ctc aag gcg ctg gcg ctg ggc ccc gac gtg agc cgc gtc tcc tcc ggc       144
   Leu Lys Ala Leu Ala Leu Gly Pro Asp Val Ser Arg Val Ser Ser Gly
           35                  40                  45
   gac gtc ctc gcc gac gcc ctc acc gac ctc cgc ctc gcg ctc aac ctc       192
   Asp Val Leu Ala Asp Ala Leu Thr Asp Leu Arg Leu Ala Leu Asn Leu
       50                  55                  60
   gac cca ctc ccg cgc cgc gcc gcc gag ggc ttc gcg ctc ttc ttc gac       240
   Asp Pro Leu Pro Arg Arg Ala Ala Glu Gly Phe Ala Leu Phe Phe Asp
   65                  70                  75                  80
   gac ctc ctg tcg cgg gcg cag gcg cgg gac tgg ttc gac cac gtc gcc       288
   Asp Leu Leu Ser Arg Ala Gln Ala Arg Asp Trp Phe Asp His Val Ala
                       85                  90                  95
   ccc tcc ctc gcc cgc ctc ctc ctc cgc ctc ccc acg ctg ctc gag ggc      336
   Pro Ser Leu Ala Arg Leu Leu Leu Arg Leu Pro Thr Leu Leu Glu Gly
                   100                 105                 110
   cac tac cgc gcc gcc ggc gac gag gct cgc ggg ctc cgc atc ctg agc      384
   His Tyr Arg Ala Ala Gly Asp Glu Ala Arg Gly Leu Arg Ile Leu Ser
               115                 120                 125
   tcg cag gat gcc ggg ctc gtg ctc ctc agc cag gag ctc gcc gcc gcg      432
   Ser Gln Asp Ala Gly Leu Val Leu Leu Ser Gln Glu Leu Ala Ala Ala
           130                 135                 140
   ctg ctc gcc tgc gcg ctc ttc tgc ctg ttc ccc acc gcc gat agg gcc      480
   Leu Leu Ala Cys Ala Leu Phe Cys Leu Phe Pro Thr Ala Asp Arg Ala
       145                 150                 155                 160
   gag gcg tgc ctc ccg gcg atc aat ttc gat agc cta ttt gcg gca ctg      528
   Glu Ala Cys Leu Pro Ala Ile Asn Phe Asp Ser Leu Phe Ala Ala Leu
                   165                 170                 175
   tgt tat aat tcg agg caa agc cag gag cag aag gtg agg tgc ctt gtt      576
   Cys Tyr Asn Ser Arg Gln Ser Gln Glu Gln Lys Val Arg Cys Leu Val
               180                 185                 190
   cac tat ttt gac agg gtg acc gct tct aca cct act ggt tcc gtt tcg      624
   His Tyr Phe Asp Arg Val Thr Ala Ser Thr Pro Thr Gly Ser Val Ser
           195                 200                 205
   ttt gag cgt aag gtt ctt cct cgc cgt cct gaa tct gat ggc att acg      672
   Phe Glu Arg Lys Val Leu Pro Arg Arg Pro Glu Ser Asp Gly Ile Thr
       210                 215                 220
   tac cct gac atg gat act tgg atg aaa tct ggt gtt ccc ctt tgc aca      720
   Tyr Pro Asp Met Asp Thr Trp Met Lys Ser Gly Val Pro Leu Cys Thr
   225                 230                 235                 240
   ttc cgg gta ttt tcc tca ggc ttg ata gaa gat gag gaa caa gaa gcc      768
   Phe Arg Val Phe Ser Ser Gly Leu Ile Glu Asp Glu Glu Gln Glu Ala
                   245                 250                 255
   ctt gaa gtt gac ttt gca aat aga tat ttg gga ggt ggc gca ctt tcc      816
   Leu Glu Val Asp Phe Ala Asn Arg Tyr Leu Gly Gly Gly Ala Leu Ser
               260                 265                 270
   aga ggc tgc gtg cag gaa gaa atc cgg ttc atg ata aac cca gaa ttg      864
   Arg Gly Cys Val Gln Glu Glu Ile Arg Phe Met Ile Asn Pro Glu Leu
           275                 280                 285
   atc gtg ggc atg ctc ttc atg gtt tca atg gaa gat aat gaa gct ata      912
   Ile Val Gly Met Leu Phe Met Val Ser Met Glu Asp Asn Glu Ala Ile
       290                 295                 300
   gaa att gtt ggt gca gaa agg ttc tca cag tac atg ggg tat ggt tcc      960
   Glu Ile Val Gly Ala Glu Arg Phe Ser Gln Tyr Met Gly Tyr Gly Ser
   305                 310                 315                 320
   tca ttc cgt ttt act ggt gac tac tta gat agc aaa ccc ttt gat gcg     1008
   Ser Phe Arg Phe Thr Gly Asp Tyr Leu Asp Ser Lys Pro Phe Asp Ala
                   325                 330                 335
   atg ggt aga cgg aaa act agg ata gtg gca att gat gct ttg gac tgt     1056
   Met Gly Arg Arg Lys Thr Arg Ile Val Ala Ile Asp Ala Leu Asp Cys
               340                 345                 350
   cca act agg tta cag ttt gaa tct agt ggt ctt cta agg gaa gtg aac     1104
   Pro Thr Arg Leu Gln Phe Glu Ser Ser Gly Leu Leu Arg Glu Val Asn
           355                 360                 365
   aag gct ttt tgt gga ttt ttg gat caa tca aat cat cag ctc tgt gca     1152
   Lys Ala Phe Cys Gly Phe Leu Asp Gln Ser Asn His Gln Leu Cys Ala
       370                 375                 380
   aag ctt gtc cag gat tta aat aca aag gat aac tgt cca agt gtc att     1200
   Lys Leu Val Gln Asp Leu Asn Thr Lys Asp Asn Cys Pro Ser Val Ile
   385                 390                 395                 400
   cct gat gaa tgc ata gga gtt tca act gga aac tgg ggt tgc ggg gct     1248
   Pro Asp Glu Cys Ile Gly Val Ser Thr Gly Asn Trp Gly Cys Gly Ala
                   405                 410                 415
   ttt ggt gga aac cct gaa atc aag agc atg att caa tgg att gct gca      1296
   Phe Gly Gly Asn Pro Glu Ile Lys Ser Met Ile Gln Trp Ile Ala Ala
               420                 425                 430
   tca cag gca ctc cga tct ttt att aac tac tac act ttt gag tcc gaa      1344
   Ser Gln Ala Leu Arg Ser Phe Ile Asn Tyr Tyr Thr Phe Glu Ser Glu
           435                 440                 445
   tca ctg aaa aga tta gaa gag gtg acc cag tgg ata ttg cgc cat agg      1392
   Ser Leu Lys Arg Leu Glu Glu Val Thr Gln Trp Ile Leu Arg His Arg
       450                 455                 460
   tgg acg gtt ggc gag ttg tgg gac atg ctt gtg gag tat tca tcc cag      1440
   Trp Thr Val Gly Glu Leu Trp Asp Met Leu Val Glu Tyr Ser Ser Gln
   465                 470                 475                 480
   agg cta aga gga gac acc aat gag ggc ttt tta aca tgg cta ctt ccc      1488
   Arg Leu Arg Gly Asp Thr Asn Glu Gly Phe Leu Thr Trp Leu Leu Pro
                   485                 490                 495
   aag gac atc ccc aat ggt gat gta gat tac atg tgt gaa tag              1530
   Lys Asp Ile Pro Asn Gly Asp Val Asp Tyr Met Cys Glu
               500                 505
<210> 16
   <211> 509
   <212> PRT
   <213> Oryza sativa
<400> 16
   Met Glu Ala Arg Gly Asp Leu Arg Ser Ile Leu Pro Tyr Leu Pro Val
   1 5 10 15
   Val Leu Arg Gly Gly Ala Leu Phe Trp Pro Pro Ala Ala Gln Glu Ala
   20 25 30
   Leu Lys Ala Leu Ala Leu Gly Pro Asp Val Ser Arg Val Ser Ser Gly
   35 40 45
   Asp Val Leu Ala Asp Ala Leu Thr Asp Leu Arg Leu Ala Leu Asn Leu
   50 55 60
   Asp Pro Leu Pro Arg Arg Ala Ala Glu Gly Phe Ala Leu Phe Phe Asp
   65 70 75 80
   Asp Leu Leu Ser Arg Ala Gln Ala Arg Asp Trp Phe Asp His Val Ala
   85 90 95
   Pro Ser Leu Ala Arg Leu Leu Leu Arg Leu Pro Thr Leu Leu Glu Gly
   100 105 110
   His Tyr Arg Ala Ala Gly Asp Glu Ala Arg Gly Leu Arg Ile Leu Ser
   115 120 125
   Ser Gln Asp Ala Gly Leu Val Leu Leu Ser Gln Glu Leu Ala Ala Ala
   130 135 140
   Leu Leu Ala Cys Ala Leu Phe Cys Leu Phe Pro Thr Ala Asp Arg Ala
   145 150 155 160
   Glu Ala Cys Leu Pro Ala Ile Asn Phe Asp Ser Leu Phe Ala Ala Leu
   165 170 175
   Cys Tyr Asn Ser Arg Gln Ser Gln Glu Gln Lys Val Arg Cys Leu Val
   180 185 190
   His Tyr Phe Asp Arg Val Thr Ala Ser Thr Pro Thr Gly Ser Val Ser
   195 200 205
   Phe Glu Arg Lys Val Leu Pro Arg Arg Pro Glu Ser Asp Gly Ile Thr
   210 215 220
   Tyr Pro Asp Met Asp Thr Trp Met Lys Ser Gly Val Pro Leu Cys Thr
   225 230 235 240
   Phe Arg Val Phe Ser Ser Gly Leu Ile Glu Asp Glu Glu Gln Glu Ala
   245 250 255
   Leu Glu Val Asp Phe Ala Asn Arg Tyr Leu Gly Gly Gly Ala Leu Ser
   260 265 270
   Arg Gly Cys Val Gln Glu Glu Ile Arg Phe Met Ile Asn Pro Glu Leu
   275 280 285
   Ile Val Gly Met Leu Phe Met Val Ser Met Glu Asp Asn Glu Ala Ile
   290 295 300
   Glu Ile Val Gly Ala Glu Arg Phe Ser Gln Tyr Met Gly Tyr Gly Ser
   305 310 315 320
   Ser Phe Arg Phe Thr Gly Asp Tyr Leu Asp Ser Lys Pro Phe Asp Ala
   325 330 335
   Met Gly Arg Arg Lys Thr Arg Ile Val Ala Ile Asp Ala Leu Asp Cys
   340 345 350
   Pro Thr Arg Leu Gln Phe Glu Ser Ser Gly Leu Leu Arg Glu Val Asn
   355 360 365
   Lys Ala Phe Cys Gly Phe Leu Asp Gln Ser Asn His Gln Leu Cys Ala
   370 375 380
   Lys Leu Val Gln Asp Leu Asn Thr Lys Asp Asn Cys Pro Ser Val Ile
   385 390 395 400
   Pro Asp Glu Cys Ile Gly Val Ser Thr Gly Asn Trp Gly Cys Gly Ala
   405 410 415
   Phe Gly Gly Asn Pro Glu Ile Lys Ser Met Ile Gln Trp Ile Ala Ala
   420 425 430
   Ser Gln Ala Leu Arg Ser Phe Ile Asn Tyr Tyr Thr Phe Glu Ser Glu
   435 440 445
   Ser Leu Lys Arg Leu Glu Glu Val Thr Gln Trp Ile Leu Arg His Arg
   450 455 460
   Trp Thr Val Gly Glu Leu Trp Asp Met Leu Val Glu Tyr Ser Ser Gln
   465 470 475 480
   Arg Leu Arg Gly Asp Thr Asn Glu Gly Phe Leu Thr Trp Leu Leu Pro
   485 490 495
   Lys Asp Ile Pro Asn Gly Asp Val Asp Tyr Met Cys Glu
   500 505
<210> 17
   <211> 25
   <212> DNA
   <213> Artificial Sequence
<220>
   <223> degenerate oligonucleotide primer PG1
   <400> 17
   atgtbccaca rmtckccrac mgtcc 25
<210> 18
   <211> 28
   <212> DNA
   <213> Artificial Sequence
<220>
   <223> degenerate oligonucleotide primer PG2
   <400> 18
   gggtytccwc caaaarcmcc rcawcccc 28
<210> 19
   <211> 26
   <212> DNA
   <213> Artificial Sequence
<220>
   <223> degenerate oligonucleotide primer PG3
   <400> 19
   gctatagaaa twgtyggtgy rgaaag 26
<210> 20
   <211> 26
   <212> DNA
   <213> Artificial Sequence
<220>
   <223> degenerate oligonucleotide primer PG4
   <400> 20
   agrggstgyg trcaggarga ratmcg 26
<210> 21
   <211> 23
   <212> DNA
   <213> Artificial Sequence
<220>
   <223> degenerate oligonucleotide primer PG5
   <220>
   <221> misc_feature
   <222> (18)..(18)
   <223> n=any nucleotide
<400> 21
   atggargaya aygargcnat hga 23
<210> 22
   <211> 24
   <212> DNA
   <213> Artificial Sequence
<220>
   <223> degenerate oligonucleotide primer PG6
<400> 22
   ccaytgdagc atrctyttda gytc 24
<210> 23
   <211> 603
   <212> DNA
   <213> zea mays
<400> 23
   tagggctgtg tgcaggagga aatccgcttc atgataaacc ccgaattgat tgtgggtatg     60
   ctattcttgt cttgtatgga agataacgag gctatagaaa tctttggtgc agaacggttc    120
   tcacagtata tgggttatgg ttcctccttt cgctttgttg gtgactattt agataccaaa    180
   ccctttgatt cgatgggcag acggagaact aggattgtgg ctatcgatgc tttggactgt    240
   ccagctaggt tacactatga atctggctgt ctcctaaggg aagtgaacaa ggcattttgt    300
   ggatttttcg atcaatcgaa acaccatctc tatgcgaagc ttttccagga tttgcacaac    360
   aaggatgact tttcaagcat caattccagt gagtacgtag gagtttcaac aggaaactgg    420
   ggttgtggtg cttttggtgg aaaccctgaa atcaagagca tgattcagtg gattgctgca    480
   tcacaggctc ttcgcccttt tgttaattac tacacttttg agaacgtgtc tctgcaaaga    540
   ttagaggagg tgatccagtg gatacggctt catggctgga ctgtcggcga gctgtggaac    600
   ata                                                                  603
<210> 24
   <211> 12987
   <212> DNA
   <213> Artificial sequence
<220>
   <223> T-DNA vector comprising a chimeric ParG expression reducing gene
   <220>
   <221> misc feature
   <222> (1)..(25)
   <223> complement of Left T-DNA border
<220>
   <221> misc_feature
   <222> (58)..(318)
   <223> complement of 3' nos
<220>
   <221> misc_feature
   <222> (337)..(888)
   <223> bar coding region
<220>
   <221> misc_feature
   <222> (889)..(1721)
   <223> 35S promoter
<220>
   <221> misc_feature
   <222> (1728)..(3123)
   <223> 35S promoter
<220>
   <221> misc_feature
   <222> (3133)..(3311)
   <223> part of ParG homologue of Zea mays
<220>
   <221> misc_feature
   <222> (3344)..(4085)
   <223> Pdk intron
<220>
   <221> misc_feature
   <222> (4119)..(4297)
   <223> part of ParG homologue of Zea mays (inverted)
<220>
   <221> misc_feature
   <222> (4310)..(5020)
   <223> 3' OCS
<220>
   <221> misc_feature
   <222> (5066)..(5042)
   <223> Right T-DNA border
<400> 24
   cggcaggata tattcaattg taaatggctc catggcgatc gctctagagg atcttcccga     60
   tctagtaaca tagatgacac cgcgcgcgat aatttatcct agtttgcgcg ctatattttg    120
   ttttctatcg cgtattaaat gtataattgc gggactctaa tcataaaaac ccatctcata    180
   aataacgtca tgcattacat gttaattatt acatgcttaa cgtaattcaa cagaaattat    240
   atgataatca tcgcaagacc ggcaacagga ttcaatctta agaaacttta ttgccaaatg    300
   tttgaacgat ctgcttcgga tcctagacgc gtgagatcag atctcggtga cgggcaggac    360
   cggacggggc ggtaccggca ggctgaagtc cagctgccag aaacccacgt catgccagtt    420
   cccgtgcttg aagccggccg cccgcagcat gccgcggggg gcatatccga gcgcctcgtg    480
   catgcgcacg ctcgggtcgt tgggcagccc gatgacagcg accacgctct tgaagccctg    540
   tgcctccagg gacttcagca ggtgggtgta gagcgtggag cccagtcccg tccgctggtg    600
   gcggggggag acgtacacgg tcgactcggc cgtccagtcg taggcgttgc gtgccttcca    660
   ggggcccgcg taggcgatgc cggcgacctc gccgtccacc tcggcgacga gccagggata    720
   gcgctcccgc agacggacga ggtcgtccgt ccactcctgc ggttcctgcg gctcggtacg    780
   gaagttgacc gtgcttgtct cgatgtagtg gttgacgatg gtgcagaccg ccggcatgtc    840
   cgcctcggtg gcacggcgga tgtcggccgg gcgtcgttct gggtccatgg ttatagagag    900
   agagatagat ttatagagag agactggtga tttcagcgtg tcctctccaa atgaaatgaa    960
   cttccttata tagaggaagg gtcttgcgaa ggatagtggg attgtgcgtc atcccttacg   1020
   tcagtggaga tgtcacatca atccacttgc tttgaagacg tggttggaac gtcttctttt   1080
   tccacgatgc tcctcgtggg tgggggtcca tctttgggac cactgtcggc agaggcatct   1140
   tgaatgatag cctttccttt atcgcaatga tggcatttgt aggagccacc ttccttttct   1200
   actgtccttt cgatgaagtg acagatagct gggcaatgga atccgaggag gtttcccgaa   1260
   attatccttt gttgaaaagt ctcaatagcc ctttggtctt ctgagactgt atctttgaca   1320
   tttttggagt agaccagagt gtcgtgctcc accatgttga cgaagatttt cttcttgtca   1380
   ttgagtcgta aaagactctg tatgaactgt tcgccagtct tcacggcgag ttctgttaga   1440
   tcctcgattt gaatcttaga ctccatgcat ggccttagat tcagtaggaa ctaccttttt   1500
   agagactcca atctctatta cttgccttgg tttatgaagc aagccttgaa tcgtccatac   1560
   tggaatagta cttctgatct tgagaaatat gtctttctct gtgttcttga tgcaattagt   1620
   cctgaatctt ttgactgcat ctttaacctt cttgggaagg tatttgatct cctggagatt   1680
   gttactcggg tagatcgtct tgatgagacc tgctgcgtag gaacgcggcc gcgtatacgt   1740
   atcgatatct tcgaattcga gctcgtcgag cggccgctcg acgaattaat tccaatccca   1800
   caaaaatctg agcttaacag cacagttgct cctctcagag cagaatcggg tattcaacac   1860
   cctcatatca actactacgt tgtgtataac ggtccacatg ccggtatata cgatgactgg   1920
   ggttgtacaa aggcggcaac aaacggcgtt cccggagttg cacacaagaa atttgccact   1980
   attacagagg caagagcagc agctgacgcg tacacaacaa gtcagcaaac agacaggttg   2040
   aacttcatcc ccaaaggaga agctcaactc aagcccaaga gctttgctaa ggccctaaca   2100
   agcccaccaa agcaaaaagc ccactggctc acgctaggaa ccaaaaggcc cagcagtgat   2160
   ccagccccaa aagagatctc ctttgccccg gagattacaa tggacgattt cctctatctt   2220
   tacgatctag gaaggaagtt cgaaggtgaa ggtgacgaca ctatgttcac cactgataat   2280
   gagaaggtta gcctcttcaa tttcagaaag aatgctgacc cacagatggt tagagaggcc   2340
   tacgcagcag gtctcatcaa gacgatctac ccgagtaaca atctccagga gatcaaatac   2400
   cttcccaaga aggttaaaga tgcagtcaaa agattcagga ctaattgcat caagaacaca   2460
   gagaaagaca tatttctcaa gatcagaagt actattccag tatggacgat tcaaggcttg   2520
   cttcataaac caaggcaagt aatagagatt ggagtctcta aaaaggtagt tcctactgaa   2580
   tctaaggcca tgcatggagt ctaagattca aatcgaggat ctaacagaac tcgccgtgaa   2640
   gactggcgaa cagttcatac agagtctttt acgactcaat gacaagaaga aaatcttcgt   2700
   caacatggtg gagcacgaca ctctggtcta ctccaaaaat gtcaaagata cagtctcaga   2760
   agaccaaagg gctattgaga cttttcaaca aaggataatt tcgggaaacc tcctcggatt   2820
   ccattgccca gctatctgtc acttcatcga aaggacagta gaaaaggaag gtggctccta   2880
   caaatgccat cattgcgata aaggaaaggc tatcattcaa gatctctctg ccgacagtgg   2940
   tcccaaagat ggacccccac ccacgaggag catcgtggaa aaagaagacg ttccaaccac   3000
   gtcttcaaag caagtggatt gatgtgacat ctccactgac gtaagggatg acgcacaatc   3060
   ccactatcct tcgcaagacc cttcctctat ataaggaagt tcatttcatt tggagaggac   3120
   acgctcgagc ccgaattgat tgtgggtatg ctattcttgt cttgtatgga agataacgag   3180
   gctatagaaa tctttggtgc agaacggttc tcacagtata tgggttatgg ttcctccttt   3240
   cgctttgttg gtgactattt agataccaaa ccctttgatt cgatgggcag acggagaact   3300
   aggattgtgg cggtacccca gcttggtaag gaaataatta ttttcttttt tccttttagt   3360
   ataaaatagt taagtgatgt taattagtat gattataata atatagttgt tataattgtg   3420
   aaaaaataat ttataaatat attgtttaca taaacaacat agtaatgtaa aaaaatatga   3480
   caagtgatgt gtaagacgaa gaagataaaa gttgagagta agtatattat ttttaatgaa   3540
   tttgatcgaa catgtaagat gatatactag cattaatatt tgttttaatc ataatagtaa   3600
   ttctagctgg tttgatgaat taaatatcaa tgataaaata ctatagtaaa aataagaata   3660
   aataaattaa aataatattt ttttatgatt aatagtttat tatataatta aatatctata   3720
   ccattactaa atattttagt ttaaaagtta ataaatattt tgttagaaat tccaatctgc   3780
   ttgtaattta tcaataaaca aaatattaaa taacaagcta aagtaacaaa taatatcaaa   3840
   ctaatagaaa cagtaatcta atgtaacaaa acataatcta atgctaatat aacaaagcgc   3900
   aagatctatc attttatata gtattatttt caatcaacat tcttattaat ttctaaataa   3960
   tacttgtagt tttattaact tctaaatgga ttgactatta attaaatgaa ttagtcgaac   4020
   atgaataaac aaggtaacat gatagatcat gtcattgtgt tatcattgat cttacatttg   4080
   gattgattac agttgggaag ctgggttcga aatcgatagc cacaatccta gttctccgtc   4140
   tgcccatcga atcaaagggt ttggtatcta aatagtcacc aacaaagcga aaggaggaac   4200
   cataacccat atactgtgag aaccgttctg caccaaagat ttctatagcc tcgttatctt   4260
   ccatacaaga caagaatagc atacccacaa tcaattcggg tctagagtcc tgctttaatg   4320
   agatatgcga gacgcctatg atcgcatgat atttgctttc aattctgttg tgcacgttgt   4380
   aaaaaacctg agcatgtgta gctcagatcc ttaccgccgg tttcggttca ttctaatgaa   4440
   tatatcaccc gttactatcg tatttttatg aataatattc tccgttcaat ttactgattg   4500
   taccctacta cttatatgta caatattaaa atgaaaacaa tatattgtgc tgaataggtt   4560
   tatagcgaca tctatgatag agcgccacaa taacaaacaa ttgcgtttta ttattacaaa   4620
   tccaatttta aaaaaagcgg cagaaccggt caaacctaaa agactgatta cataaatctt   4680
   attcaaattt caaaaggccc caggggctag tatctacgac acaccgagcg gcgaactaat   4740
   aacgttcact gaagggaact ccggttcccc gccggcgcgc atgggtgaga ttccttgaag   4800
   ttgagtattg gccgtccgct ctaccgaaag ttacgggcac cattcaaccc ggtccagcac   4860
   ggcggccggg taaccgactt gctgccccga gaattatgca gcattttttt ggtgtatgtg   4920
   ggccccaaat gaagtgcagg tcaaaccttg acagtgacga caaatcgttg ggcgggtcca   4980
   gggcgaattt tgcgacaaca tgtcgaggct cagcaggacc tgcaggtcga cggccgagta   5040
   ctggcaggat atataccgtt gtaatttgtc gcgtgtgaat aagtcgctgt gtatgtttgt   5100
   ttgattgttt ctgttggagt gcagcccatt tcaccggaca agtcggctag attgatttag   5160
   ccctgatgaa ctgccgaggg gaagccatct tgagcgcgga atgggaatgg atttcgttgt   5220
   acaacgagac gacagaacac ccacgggacc gagcttcgat cgagcatcaa atgaaactgc   5280
   aatttattca tatcaggatt atcaatacca tatttttgaa aaagccgttt ctgtaatgaa   5340
   ggagaaaact caccgaggca gttccatagg atggcaagat cctggtatcg gtctgcgatt   5400
   ccgactcgtc caacatcaat acaacctatt aatttcccct cgtcaaaaat aaggttatca   5460
   agtgagaaat caccatgagt gacgactgaa tccggtgaga atggcaaaag tttatgcatt   5520
   tctttccaga cttgttcaac aggccagcca ttacgctcgt catcaaaatc actcgcatca   5580
   accaaaccgt tattcattcg tgattgcgcc tgagcgagac gaaatacgcc gctgttaaaa   5640
   ggacaattac aaacaggaat cgaatgcaac cggcgcagga acactgccag cgcatcaaca   5700
   atattttcac ctgaatcagg atattcttct aatacctgga atgctgtttt tccggggatc   5760
   gcagtggtga gtaaccatgc atcatcagga gtacggataa aatgcttgat ggtcggaaga   5820
   ggcataaatt ccgtcagcca gtttagtctg accatctcat ctgtaacatc attggcaacg   5880
   ctacctttgc catgtttcag aaacaactct ggcgcatcgg gcttcccata caatcgatag   5940
   attgtcgcac ctgattgccc gacattatcc gaatctggca attccggttc gcttgctgtc   6000
   cataaaaccg cccagtctag ctatcgccat gtaagcccac tgcaagctac ctgctttctc   6060
   tttgcgcttg cgttttccgg atcttcttga gatccttttt ttctgcgcgt aatctgctgc   6120
   ttgcaaacaa aaaaaccacc gctaccagcg gtggtttgtt tgccggatca agagctacca   6180
   actctttttc cgaaggtaac tggcttcagc agagcgcaga taccaaatac tgtccttcta   6240
   gtgtagccgt agttaggcca ccacttcaag aactctgtag caccgcctac atacctcgct 6300
   ctgctaatcc tgttaccagt ggctgctgcc agtggcgata agtcgtgtct taccgggttg 6360
   gactcaagac gatagttacc ggataaggcg cagcggtcgg gctgaacggg gggttcgtgc 6420
   acacagccca gcttggagcg aacgacctac accgaactga gatacctaca gcgtgagcta 6480
   tgagaaagcg ccacgcttcc cgaagggaga aaggcggaca ggtatccggt aagcggcagg 6540
   gtcggaacag gagagcgcac gagggagctt ccagggggaa acgcctggta tctttatagt 6600
   cctgtcgggt ttcgccacct ctgacttgag cgtcgatttt tgtgatgctc gtcagggggg 6660
   cggagcctat ggaaaaacgc cagcaacgcg gcctttttac ggttcctggc cttttgctgg 6720
   ccttttgctc acatgttctt tcctgcgtta tcccctgatt ctgtggataa ccgtattacc 6780
   gcctttgagt gagctgatac cgctcgccgc agccgaacga ccgagcgcag cgagtcagtg 6840
   agcgaggaag cggaagagcg cctgatgcgg tattttctcc ttacgcatct gtgcggtatt 6900
   tcacaccgca tatggtgcac tctcagtaca atctgctctg atgccgcata gttaagccag 6960
   tatacactcc gctatcgcta cgtgactggg tcatggctgc gccccgacac ccgccaacac 7020
   ccgctgacgc gccctgacgg gcttgtctgc tcccggcatc cgcttacaga caagctgtga 7080
   ccgtctccgg gagctgcatg tgtcagaggt tttcaccgtc atcaccgaaa cgcgcgaggc 7140
   agggtgcctt gatgtgggcg ccggcggtcg agtggcgacg gcgcggcttg tccgcgccct 7200
   ggtagattgc ctggccgtag gccagccatt tttgagcggc cagcggccgc gataggccga 7260
   cgcgaagcgg cggggcgtag ggagcgcagc gaccgaaggg taggcgcttt ttgcagctct 7320
   tcggctgtgc gctggccaga cagttatgca caggccaggc gggttttaag agttttaata 7380
   agttttaaag agttttaggc ggaaaaatcg ccttttttct cttttatatc agtcacttac 7440
   atgtgtgacc ggttcccaat gtacggcttt gggttcccaa tgtacgggtt ccggttccca 7500
   atgtacggct ttgggttccc aatgtacgtg ctatccacag gaaagagacc ttttcgacct 7560
   ttttcccctg ctagggcaat ttgccctagc atctgctccg tacattagga accggcggat 7620
   gcttcgccct cgatcaggtt gcggtagcgc atgactagga tcgggccagc ctgccccgcc 7680
   tcctccttca aatcgtactc cggcaggtca tttgacccga tcagcttgcg cacggtgaaa 7740
   cagaacttct tgaactctcc ggcgctgcca ctgcgttcgt agatcgtctt gaacaaccat 7800
   ctggcttctg ccttgcctgc ggcgcggcgt gccaggcggt agagaaaacg gccgatgccg 7860
   ggatcgatca aaaagtaatc ggggtgaacc gtcagcacgt ccgggttctt gccttctgtg 7920
   atctcgcggt acatccaatc agctagctcg atctcgatgt actccggccg cccggtttcg 7980
   ctctttacga tcttgtagcg gctaatcaag gcttcaccct cggataccgt caccaggcgg 8040
   ccgttcttgg ccttcttcgt acgctgcatg gcaacgtgcg tggtgtttaa ccgaatgcag 8100
   gtttctacca ggtcgtcttt ctgctttccg ccatcggctc gccggcagaa cttgagtacg 8160
   tccgcaacgt gtggacggaa cacgcggccg ggcttgtctc ccttcccttc ccggtatcgg 8220
   ttcatggatt cggttagatg ggaaaccgcc atcagtacca ggtcgtaatc ccacacactg 8280
   gccatgccgg ccggccctgc ggaaacctct acgtgcccgt ctggaagctc gtagcggatc 8340
   acctcgccag ctcgtcggtc acgcttcgac agacggaaaa cggccacgtc catgatgctg 8400
   cgactatcgc gggtgcccac gtcatagagc atcggaacga aaaaatctgg ttgctcgtcg 8460
   cccttgggcg gcttcctaat cgacggcgca ccggctgccg gcggttgccg ggattctttg 8520
   cggattcgat cagcggccgc ttgccacgat tcaccggggc gtgcttctgc ctcgatgcgt 8580
   tgccgctggg cggcctgcgc ggccttcaac ttctccacca ggtcatcacc cagcgccgcg 8640
   ccgatttgta ccgggccgga tggtttgcga ccgtcacgcc gattcctcgg gcttgggggt 8700
   tccagtgcca ttgcagggcc ggcagacaac ccagccgctt acgcctggcc aaccgcccgt 8760
   tcctccacac atggggcatt ccacggcgtc ggtgcctggt tgttcttgat tttccatgcc 8820
   gcctccttta gccgctaaaa ttcatctact catttattca tttgctcatt tactctggta 8880
   gctgcgcgat gtattcagat agcagctcgg taatggtctt gccttggcgt accgcgtaca 8940
   tcttcagctt ggtgtgatcc tccgccggca actgaaagtt gacccgcttc atggctggcg 9000
   tgtctgccag gctggccaac gttgcagcct tgctgctgcg tgcgctcgga cggccggcac 9060
   ttagcgtgtt tgtgcttttg ctcattttct ctttacctca ttaactcaaa tgagttttga 9120
   tttaatttca gcggccagcg cctggacctc gcgggcagcg tcgccctcgg gttctgattc 9180
   aagaacggtt gtgccggcgg cggcagtgcc tgggtagctc acgcgctgcg tgatacggga 9240
   ctcaagaatg ggcagctcgt acccggccag cgcctcggca acctcaccgc cgatgcgcgt 9300
   gcctttgatc gcccgcgaca cgacaaaggc cgcttgtagc cttccatccg tgacctcaat 9360
   gcgctgctta accagctcca ccaggtcggc ggtggcccat atgtcgtaag ggcttggctg 9420
   caccggaatc agcacgaagt cggctgcctt gatcgcggac acagccaagt ccgccgcctg 9480
   gggcgctccg tcgatcacta cgaagtcgcg ccggccgatg gccttcacgt cgcggtcaat 9540
   cgtcgggcgg tcgatgccga caacggttag cggttgatct tcccgcacgg ccgcccaatc 9600
   gcgggcactg ccctggggat cggaatcgac taacagaaca tcggccccgg cgagttgcag 9660
   ggcgcgggct agatgggttg cgatggtcgt cttgcctgac ccgcctttct ggttaagtac 9720
   agcgataacc ttcatgcgtt ccccttgcgt atttgtttat ttactcatcg catcatatac 9780
   gcagcgaccg catgacgcaa gctgttttac tcaaatacac atcacctttt tagacggcgg 9840
   cgctcggttt cttcagcggc caagctggcc ggccaggccg ccagcttggc atcagacaaa 9900
   ccggccagga tttcatgcag ccgcacggtt gagacgtgcg cgggcggctc gaacacgtac 9960
   ccggccgcga tcatctccgc ctcgatctct tcggtaatga aaaacggttc gtcctggccg 10020
   tcctggtgcg gtttcatgct tgttcctctt ggcgttcatt ctcggcggcc gccagggcgt 10080
   cggcctcggt caatgcgtcc tcacggaagg caccgcgccg cctggcctcg gtgggcgtca 10140
   cttcctcgct gcgctcaagt gcgcggtaca gggtcgagcg atgcacgcca agcagtgcag 10200
   ccgcctcttt cacggtgcgg ccttcctggt cgatcagctc gcgggcgtgc gcgatctgtg 10260
   ccggggtgag ggtagggcgg gggccaaact tcacgcctcg ggccttggcg gcctcgcgcc 10320
   cgctccgggt gcggtcgatg attagggaac gctcgaactc ggcaatgccg gcgaacacgg 10380
   tcaacaccat gcggccggcc ggcgtggtgg tgtcggccca cggctctgcc aggctacgca 10440
   ggcccgcgcc ggcctcctgg atgcgctcgg caatgtccag taggtcgcgg gtgctgcggg 10500
   ccaggcggtc tagcctggtc actgtcacaa cgtcgccagg gcgtaggtgg tcaagcatcc 10560
   tggccagctc cgggcggtcg cgcctggtgc cggtgatctt ctcggaaaac agcttggtgc 10620
   agccggccgc gtgcagttcg gcccgttggt tggtcaagtc ctggtcgtcg gtgctgacgc 10680
   gggcatagcc cagcaggcca gcggcggcgc tcttgttcat ggcgtaatgt ctccggttct 10740
   agtcgcaagt attctacttt atgcgactaa aacacgcgac aagaaaacgc caggaaaagg 10800
   gcagggcggc agcctgtcgc gtaacttagg acttgtgcga catgtcgttt tcagaagacg 10860
   gctgcactga acgtcagaag ccgactgcac tatagcagcg gaggggttgg atcgatccct 10920
   gctcgcgcag gctgggtgcc aagctctcgg gtaacatcaa ggcccgatcc ttggagccct 10980
   tgccctcccg cacgatgatc gtgccgtgat cgaaatccag atccttgacc cgcagttgca 11040
   aaccctcact gatccgcatg cccgttccat acagaagctg ggcgaacaaa cgatgctcgc 11100
   cttccagaaa accgaggatg cgaaccactt catccggggt cagcaccacc ggcaagcgcc 11160
   cggacggccg aggtcttccg atctcctgaa gccagggcag atccgtgcac agcacttgcc 11220
   gtagaagaac agcaaggccg ccaatgcctg acgatgcgtg gagaccgaaa ccttgcgctc 11280
   gttcgccagc caggacagaa atgcctcgac ttcgctgctg cccaaggttg ccgggtgacg 11340
   cacaccgtgg aaacggatga aggcacgaac ccagtggaca taagcctgtt cggttcgtaa 11400
   gctgtaatgc aagtagcgta tgcgctcacg caactggtcc agaaccttga ccgaacgcag 11460
   cggtggtaac ggcgcagtgg cggttttcat ggcttgttat gactgttttt ttggggtaca 11520
   gtctatgcct cgggcatcca agcagcaagc gcgttacgcc gtgggtcgat gtttgatgtt 11580
   atggagcagc aacgatgtta cgcagcaggg cagtcgccct aaaacaaagt taaacatcat 11640
   gagggaagcg gtgatcgccg aagtatcgac tcaactatca gaggtagttg gcgtcatcga 11700
   gcgccatctc gaaccgacgt tgctggccgt acatttgtac ggctccgcag tggatggcgg 11760
   cctgaagcca cacagtgata ttgatttgct ggttacggtg accgtaaggc ttgatgaaac 11820
   aacgcggcga gctttgatca acgacctttt ggaaacttcg gcttcccctg gagagagcga 11880
   gattctccgc gctgtagaag tcaccattgt tgtgcacgac gacatcattc cgtggcgtta 11940
   tccagctaag cgcgaactgc aatttggaga atggcagcgc aatgacattc ttgcaggtat 12000
   cttcgagcca gccacgatcg acattgatct ggctatcttg ctgacaaaag caagagaaca 12060
   tagcgttgcc ttggtaggtc cagcggcgga ggaactcttt gatccggttc ctgaacagga 12120
   tctatttgag gcgctaaatg aaaccttaac gctatggaac tcgccgcccg actgggctgg 12180
   cgatgagcga aatgtagtgc ttacgttgtc ccgcatttgg tacagcgcag taaccggcaa 12240
   aatcgcgccg aaggatgtcg ctgccgactg ggcaatggag cgcctgccgg cccagtatca 12300
   gcccgtcata cttgaagcta gacaggctta tcttggacaa gaagaagatc gcttggcctc 12360
   gcgcgcagat cagttggaag aatttgtcca ctacgtgaaa ggcgagatca ccaaggtagt 12420
   cggcaaataa tgtctaacaa ttcgttcaag ccgacgccgc ttcgcggcgc ggcttaactc 12480
   aagcgttaga tgcactaagc acataattgc tcacagccaa actatcaggt caagtctgct 12540
   tttattattt ttaagcgtgc ataataagcc ctacacaaat tgggagatat atcatgaaag 12600
   gctggctttt tcttgttatc gcaatagttg gcgaagtaat cgcaacatcc gcattaaaat 12660
   ctagcgaggg ctttactaag ctagcttgct tggtcgttcc ggtaccgtga acgtcggctc 12720
   gattgtacct gcgttcaaat actttgcgat cgtgttgcgc gcctgcccgg tgcgtcggct 12780
   gatctcacgg atcgactgct tctctcgcaa cgccatccga cggatgatgt ttaaaagtcc 12840
   catgtggatc actccgttgc cccgtcgctc accgtgttgg ggggaaggtg cacatggctc 12900
   agttctcaat ggaaattatc tgcctaaccg gctcagttct gcgtagaaac caacatgcaa 12960
   gctccaccgg gtgcaaagcg gcagcgg 12987
<210> 25
   <211> 13226
   <212> DNA
   <213> Artificial sequence
<220>
   <223> T-DNA vector comprising a chimeric ParG expression reducing gene
   <220>
   <221> misc_feature
   <222> (1)..(25)
   <223> Left T-DNA border (C)
<220>
   <221> misc_feature
   <222> (58)..(318)
   <223> 3' nos (C)
<220>
   <221> misc_feature
   <222> (337)..(888)
   <223> bar coding region (C)
<220>
   <221> misc_feature
   <222> (889)..(1721)
   <223> 35S3 promoter region (C)
<220>
   <221> misc_feature
   <222> (1778)..(3123)
   <223> 35S promoter region
<220>
   <221> misc_feature
   <222> (3130)..(3431)
   <223> part of ParG homologue of Zea mays
<220>
   <221> misc_feature
   <222> (3464)..(4205)
   <223> Pdk-intron
<220>
   <221> misc_feature
   <222> (4238)..(4536)
   <223> part of ParG homologue of Zea mays (C)
<220>
   <221> misc_feature
   <222> (4549)..(5259)
   <223> 3' ocs
<220>
   <221> misc_feature
   <222> (5281)..(5305)
   <223> Right T-dNA border (C)
<400> 25
   cggcaggata tattcaattg taaatggctc catggcgatc gctctagagg atcttcccga     60
   tctagtaaca tagatgacac cgcgcgcgat aatttatcct agtttgcgcg ctatattttg    120
   ttttctatcg cgtattaaat gtataattgc gggactctaa tcataaaaac ccatctcata    180
   aataacgtca tgcattacat gttaattatt acatgcttaa cgtaattcaa cagaaattat    240
   atgataatca tcgcaagacc ggcaacagga ttcaatctta agaaacttta ttgccaaatg    300
   tttgaacgat ctgcttcgga tcctagacgc gtgagatcag atctcggtga cgggcaggac    360
   cggacggggc ggtaccggca ggctgaagtc cagctgccag aaacccacgt catgccagtt    420
   cccgtgcttg aagccggccg cccgcagcat gccgcggggg gcatatccga gcgcctcgtg    480
   catgcgcacg ctcgggtcgt tgggcagccc gatgacagcg accacgctct tgaagccctg    540
   tgcctccagg gacttcagca ggtgggtgta gagcgtggag cccagtcccg tccgctggtg    600
   gcggggggag acgtacacgg tcgactcggc cgtccagtcg taggcgttgc gtgccttcca    660
   ggggcccgcg taggcgatgc cggcgacctc gccgtccacc tcggcgacga gccagggata     720
   gcgctcccgc agacggacga ggtcgtccgt ccactcctgc ggttcctgcg gctcggtacg     780
   gaagttgacc gtgcttgtct cgatgtagtg gttgacgatg gtgcagaccg ccggcatgtc     840
   cgcctcggtg gcacggcgga tgtcggccgg gcgtcgttct gggtccatgg ttatagagag     900
   agagatagat ttatagagag agactggtga tttcagcgtg tcctctccaa atgaaatgaa     960
   cttccttata tagaggaagg gtcttgcgaa ggatagtggg attgtgcgtc atcccttacg    1020
   tcagtggaga tgtcacatca atccacttgc tttgaagacg tggttggaac gtcttctttt    1080
   tccacgatgc tcctcgtggg tgggggtcca tctttgggac cactgtcggc agaggcatct    1140
   tgaatgatag cctttccttt atcgcaatga tggcatttgt aggagccacc ttccttttct    1200
   actgtccttt cgatgaagtg acagatagct gggcaatgga atccgaggag gtttcccgaa    1260
   attatccttt gttgaaaagt ctcaatagcc ctttggtctt ctgagactgt atctttgaca    1320
   tttttggagt agaccagagt gtcgtgctcc accatgttga cgaagatttt cttcttgtca    1380
   ttgagtcgta aaagactctg tatgaactgt tcgccagtct tcacggcgag ttctgttaga    1440
   tcctcgattt gaatcttaga ctccatgcat ggccttagat tcagtaggaa ctaccttttt    1500
   agagactcca atctctatta cttgccttgg tttatgaagc aagccttgaa tcgtccatac    1560
   tggaatagta cttctgatct tgagaaatat gtctttctct gtgttcttga tgcaattagt    1620
   cctgaatctt ttgactgcat ctttaacctt cttgggaagg tatttgatct cctggagatt    1680
   gttactcggg tagatcgtct tgatgagacc tgctgcgtag gaacgcggcc gcgtatacgt    1740
   atcgatatct tcgaattcga gctcgtcgag cggccgctcg acgaattaat tccaatccca    1800
   caaaaatctg agcttaacag cacagttgct cctctcagag cagaatcggg tattcaacac    1860
   cctcatatca actactacgt tgtgtataac ggtccacatg ccggtatata cgatgactgg    1920
   ggttgtacaa aggcggcaac aaacggcgtt cccggagttg cacacaagaa atttgccact    1980
   attacagagg caagagcagc agctgacgcg tacacaacaa gtcagcaaac agacaggttg    2040
   aacttcatcc ccaaaggaga agctcaactc aagcccaaga gctttgctaa ggccctaaca    2100
   agcccaccaa agcaaaaagc ccactggctc acgctaggaa ccaaaaggcc cagcagtgat    2160
   ccagccccaa aagagatctc ctttgccccg gagattacaa tggacgattt cctctatctt    2220
   tacgatctag gaaggaagtt cgaaggtgaa ggtgacgaca ctatgttcac cactgataat    2280
   gagaaggtta gcctcttcaa tttcagaaag aatgctgacc cacagatggt tagagaggcc    2340
   tacgcagcag gtctcatcaa gacgatctac ccgagtaaca atctccagga gatcaaatac    2400
   cttcccaaga aggttaaaga tgcagtcaaa agattcagga ctaattgcat caagaacaca    2460
   gagaaagaca tatttctcaa gatcagaagt actattccag tatggacgat tcaaggcttg    2520
   cttcataaac caaggcaagt aatagagatt ggagtctcta aaaaggtagt tcctactgaa    2580
   tctaaggcca tgcatggagt ctaagattca aatcgaggat ctaacagaac tcgccgtgaa    2640
   gactggcgaa cagttcatac agagtctttt acgactcaat gacaagaaga aaatcttcgt    2700
   caacatggtg gagcacgaca ctctggtcta ctccaaaaat gtcaaagata cagtctcaga    2760
   agaccaaagg gctattgaga cttttcaaca aaggataatt tcgggaaacc tcctcggatt    2820
   ccattgccca gctatctgtc acttcatcga aaggacagta gaaaaggaag gtggctccta    2880
   caaatgccat cattgcgata aaggaaaggc tatcattcaa gatctctctg ccgacagtgg    2940
   tcccaaagat ggacccccac ccacgaggag catcgtggaa aaagaagacg ttccaaccac    3000
   gtcttcaaag caagtggatt gatgtgacat ctccactgac gtaagggatg acgcacaatc    3060
   ccactatcct tcgcaagacc cttcctctat ataaggaagt tcatttcatt tggagaggac    3120
   acgctcgagg aatctggctg tctcctaagg gaagtgaaca aggcattttg tggatttttc    3180
   gatcaatcga aacaccatct ctatgcgaag cttttccagg atttgcacaa caaggatgac    3240
   ttttcaagca tcaattccag tgagtacgta ggagtttcaa caggaaactg gggttgtggt    3300
   gcttttggtg gaaaccctga aatcaagagc atgattcagt ggattgctgc atcacaggct    3360
   cttcgccctt ttgttaatta ctacactttt gagaacgtgt ctctgcaaag attagaggag    3420
   gtgatccagt gggtacccca gcttggtaag gaaataatta ttttcttttt tccttttagt    3480
   ataaaatagt taagtgatgt taattagtat gattataata atatagttgt tataattgtg    3540
   aaaaaataat ttataaatat attgtttaca taaacaacat agtaatgtaa aaaaatatga    3600
   caagtgatgt gtaagacgaa gaagataaaa gttgagagta agtatattat ttttaatgaa    3660
   tttgatcgaa catgtaagat gatatactag cattaatatt tgttttaatc ataatagtaa    3720
   ttctagctgg tttgatgaat taaatatcaa tgataaaata ctatagtaaa aataagaata    3780
   aataaattaa aataatattt ttttatgatt aatagtttat tatataatta aatatctata    3840
   ccattactaa atattttagt ttaaaagtta ataaatattt tgttagaaat tccaatctgc    3900
   ttgtaattta tcaataaaca aaatattaaa taacaagcta aagtaacaaa taatatcaaa    3960
   ctaatagaaa cagtaatcta atgtaacaaa acataatcta atgctaatat aacaaagcgc    4020
   aagatctatc attttatata gtattatttt caatcaacat tcttattaat ttctaaataa    4080
   tacttgtagt tttattaact tctaaatgga ttgactatta attaaatgaa ttagtcgaac    4140
   atgaataaac aaggtaacat gatagatcat gtcattgtgt tatcattgat cttacatttg    4200
   gattgattac agttgggaag ctgggttcga aatcgatcac tggatcacct cctctaatct    4260
   ttgcagagac acgttctcaa aagtgtagta attaacaaaa gggcgaagag cctgtgatgc    4320
   agcaatccac tgaatcatgc tcttgatttc agggtttcca ccaaaagcac cacaacccca 4380
   gtttcctgtt gaaactccta cgtactcact ggaattgatg cttgaaaagt catccttgtt 4440
   gtgcaaatcc tggaaaagct tcgcatagag atggtgtttc gattgatcga aaaatccaca 4500
   aaatgccttg ttcacttccc ttaggagaca gccagattct ctagagtcct gctttaatga 4560
   gatatgcgag acgcctatga tcgcatgata tttgctttca attctgttgt gcacgttgta 4620
   aaaaacctga gcatgtgtag ctcagatcct taccgccggt ttcggttcat tctaatgaat 4680
   atatcacccg ttactatcgt atttttatga ataatattct ccgttcaatt tactgattgt 4740
   accctactac ttatatgtac aatattaaaa tgaaaacaat atattgtgct gaataggttt 4800
   atagcgacat ctatgataga gcgccacaat aacaaacaat tgcgttttat tattacaaat 4860
   ccaattttaa aaaaagcggc agaaccggtc aaacctaaaa gactgattac ataaatctta 4920
   ttcaaatttc aaaaggcccc aggggctagt atctacgaca caccgagcgg cgaactaata 4980
   acgttcactg aagggaactc cggttccccg ccggcgcgca tgggtgagat tccttgaagt 5040
   tgagtattgg ccgtccgctc taccgaaagt tacgggcacc attcaacccg gtccagcacg 5100
   gcggccgggt aaccgacttg ctgccccgag aattatgcag catttttttg gtgtatgtgg 5160
   gccccaaatg aagtgcaggt caaaccttga cagtgacgac aaatcgttgg gcgggtccag 5220
   ggcgaatttt gcgacaacat gtcgaggctc agcaggacct gcaggtcgac ggccgagtac 5280
   tggcaggata tataccgttg taatttgtcg cgtgtgaata agtcgctgtg tatgtttgtt 5340
   tgattgtttc tgttggagtg cagcccattt caccggacaa gtcggctaga ttgatttagc 5400
   cctgatgaac tgccgagggg aagccatctt gagcgcggaa tgggaatgga tttcgttgta 5460
   caacgagacg acagaacacc cacgggaccg agcttcgatc gagcatcaaa tgaaactgca 5520
   atttattcat atcaggatta tcaataccat atttttgaaa aagccgtttc tgtaatgaag 5580
   gagaaaactc accgaggcag ttccatagga tggcaagatc ctggtatcgg tctgcgattc 5640
   cgactcgtcc aacatcaata caacctatta atttcccctc gtcaaaaata aggttatcaa 5700
   gtgagaaatc accatgagtg acgactgaat ccggtgagaa tggcaaaagt ttatgcattt 5760
   ctttccagac ttgttcaaca ggccagccat tacgctcgtc atcaaaatca ctcgcatcaa 5820
   ccaaaccgtt attcattcgt gattgcgcct gagcgagacg aaatacgccg ctgttaaaag 5880
   gacaattaca aacaggaatc gaatgcaacc ggcgcaggaa cactgccagc gcatcaacaa 5940
   tattttcacc tgaatcagga tattcttcta atacctggaa tgctgttttt ccggggatcg 6000
   cagtggtgag taaccatgca tcatcaggag tacggataaa atgcttgatg gtcggaagag 6060
   gcataaattc cgtcagccag tttagtctga ccatctcatc tgtaacatca ttggcaacgc 6120
   tacctttgcc atgtttcaga aacaactctg gcgcatcggg cttcccatac aatcgataga 6180
   ttgtcgcacc tgattgcccg acattatccg aatctggcaa ttccggttcg cttgctgtcc 6240
   ataaaaccgc ccagtctagc tatcgccatg taagcccact gcaagctacc tgctttctct 6300
   ttgcgcttgc gttttccgga tcttcttgag atcctttttt tctgcgcgta atctgctgct 6360
   tgcaaacaaa aaaaccaccg ctaccagcgg tggtttgttt gccggatcaa gagctaccaa 6420
   ctctttttcc gaaggtaact ggcttcagca gagcgcagat accaaatact gtccttctag 6480
   tgtagccgta gttaggccac cacttcaaga actctgtagc accgcctaca tacctcgctc 6540
   tgctaatcct gttaccagtg gctgctgcca gtggcgataa gtcgtgtctt accgggttgg 6600
   actcaagacg atagttaccg gataaggcgc agcggtcggg ctgaacgggg ggttcgtgca 6660
   cacagcccag cttggagcga acgacctaca ccgaactgag atacctacag cgtgagctat 6720
   gagaaagcgc cacgcttccc gaagggagaa aggcggacag gtatccggta agcggcaggg 6780
   tcggaacagg agagcgcacg agggagcttc cagggggaaa cgcctggtat ctttatagtc 6840
   ctgtcgggtt tcgccacctc tgacttgagc gtcgattttt gtgatgctcg tcaggggggc 6900
   ggagcctatg gaaaaacgcc agcaacgcgg cctttttacg gttcctggcc ttttgctggc 6960
   cttttgctca catgttcttt cctgcgttat cccctgattc tgtggataac cgtattaccg 7020
   cctttgagtg agctgatacc gctcgccgca gccgaacgac cgagcgcagc gagtcagtga 7080
   gcgaggaagc ggaagagcgc ctgatgcggt attttctcct tacgcatctg tgcggtattt 7140
   cacaccgcat atggtgcact ctcagtacaa tctgctctga tgccgcatag ttaagccagt 7200
   atacactccg ctatcgctac gtgactgggt catggctgcg ccccgacacc cgccaacacc 7260
   cgctgacgcg ccctgacggg cttgtctgct cccggcatcc gcttacagac aagctgtgac 7320
   cgtctccggg agctgcatgt gtcagaggtt ttcaccgtca tcaccgaaac gcgcgaggca 7380
   gggtgccttg atgtgggcgc cggcggtcga gtggcgacgg cgcggcttgt ccgcgccctg 7440
   gtagattgcc tggccgtagg ccagccattt ttgagcggcc agcggccgcg ataggccgac 7500
   gcgaagcggc ggggcgtagg gagcgcagcg accgaagggt aggcgctttt tgcagctctt 7560
   cggctgtgcg ctggccagac agttatgcac aggccaggcg ggttttaaga gttttaataa 7620
   gttttaaaga gttttaggcg gaaaaatcgc cttttttctc ttttatatca gtcacttaca 7680
   tgtgtgaccg gttcccaatg tacggctttg ggttcccaat gtacgggttc cggttcccaa 7740
   tgtacggctt tgggttccca atgtacgtgc tatccacagg aaagagacct tttcgacctt 7800
   tttcccctgc tagggcaatt tgccctagca tctgctccgt acattaggaa ccggcggatg 7860
   cttcgccctc gatcaggttg cggtagcgca tgactaggat cgggccagcc tgccccgcct 7920
   cctccttcaa atcgtactcc ggcaggtcat ttgacccgat cagcttgcgc acggtgaaac 7980
   agaacttctt gaactctccg gcgctgccac tgcgttcgta gatcgtcttg aacaaccatc 8040
   tggcttctgc cttgcctgcg gcgcggcgtg ccaggcggta gagaaaacgg ccgatgccgg 8100
   gatcgatcaa aaagtaatcg gggtgaaccg tcagcacgtc cgggttcttg ccttctgtga 8160
   tctcgcggta catccaatca gctagctcga tctcgatgta ctccggccgc ccggtttcgc 8220
   tctttacgat cttgtagcgg ctaatcaagg cttcaccctc ggataccgtc accaggcggc 8280
   cgttcttggc cttcttcgta cgctgcatgg caacgtgcgt ggtgtttaac cgaatgcagg 8340
   tttctaccag gtcgtctttc tgctttccgc catcggctcg ccggcagaac ttgagtacgt 8400
   ccgcaacgtg tggacggaac acgcggccgg gcttgtctcc cttcccttcc cggtatcggt 8460
   tcatggattc ggttagatgg gaaaccgcca tcagtaccag gtcgtaatcc cacacactgg 8520
   ccatgccggc cggccctgcg gaaacctcta cgtgcccgtc tggaagctcg tagcggatca 8580
   cctcgccagc tcgtcggtca cgcttcgaca gacggaaaac ggccacgtcc atgatgctgc 8640
   gactatcgcg ggtgcccacg tcatagagca tcggaacgaa aaaatctggt tgctcgtcgc 8700
   ccttgggcgg cttcctaatc gacggcgcac cggctgccgg cggttgccgg gattctttgc 8760
   ggattcgatc agcggccgct tgccacgatt caccggggcg tgcttctgcc tcgatgcgtt 8820
   gccgctgggc ggcctgcgcg gccttcaact tctccaccag gtcatcaccc agcgccgcgc 8880
   cgatttgtac cgggccggat ggtttgcgac cgtcacgccg attcctcggg cttgggggtt 8940
   ccagtgccat tgcagggccg gcagacaacc cagccgctta cgcctggcca accgcccgtt 9000
   cctccacaca tggggcattc cacggcgtcg gtgcctggtt gttcttgatt ttccatgccg 9060
   cctcctttag ccgctaaaat tcatctactc atttattcat ttgctcattt actctggtag 9120
   ctgcgcgatg tattcagata gcagctcggt aatggtcttg ccttggcgta ccgcgtacat 9180
   cttcagcttg gtgtgatcct ccgccggcaa ctgaaagttg acccgcttca tggctggcgt 9240
   gtctgccagg ctggccaacg ttgcagcctt gctgctgcgt gcgctcggac ggccggcact 9300
   tagcgtgttt gtgcttttgc tcattttctc tttacctcat taactcaaat gagttttgat 9360
   ttaatttcag cggccagcgc ctggacctcg cgggcagcgt cgccctcggg ttctgattca 9420
   agaacggttg tgccggcggc ggcagtgcct gggtagctca cgcgctgcgt gatacgggac 9480
   tcaagaatgg gcagctcgta cccggccagc gcctcggcaa cctcaccgcc gatgcgcgtg 9540
   cctttgatcg cccgcgacac gacaaaggcc gcttgtagcc ttccatccgt gacctcaatg 9600
   cgctgcttaa ccagctccac caggtcggcg gtggcccata tgtcgtaagg gcttggctgc 9660
   accggaatca gcacgaagtc ggctgccttg atcgcggaca cagccaagtc cgccgcctgg 9720
   ggcgctccgt cgatcactac gaagtcgcgc cggccgatgg ccttcacgtc gcggtcaatc 9780
   gtcgggcggt cgatgccgac aacggttagc ggttgatctt cccgcacggc cgcccaatcg 9840
   cgggcactgc cctggggatc ggaatcgact aacagaacat cggccccggc gagttgcagg 9900
   gcgcgggcta gatgggttgc gatggtcgtc ttgcctgacc cgcctttctg gttaagtaca 9960
   gcgataacct tcatgcgttc cccttgcgta tttgtttatt tactcatcgc atcatatacg 10020
   cagcgaccgc atgacgcaag ctgttttact caaatacaca tcaccttttt agacggcggc 10080
   gctcggtttc ttcagcggcc aagctggccg gccaggccgc cagcttggca tcagacaaac 10140
   cggccaggat ttcatgcagc cgcacggttg agacgtgcgc gggcggctcg aacacgtacc 10200
   cggccgcgat catctccgcc tcgatctctt cggtaatgaa aaacggttcg tcctggccgt 10260
   cctggtgcgg tttcatgctt gttcctcttg gcgttcattc tcggcggccg ccagggcgtc 10320
   ggcctcggtc aatgcgtcct cacggaaggc accgcgccgc ctggcctcgg tgggcgtcac 10380
   ttcctcgctg cgctcaagtg cgcggtacag ggtcgagcga tgcacgccaa gcagtgcagc 10440
   cgcctctttc acggtgcggc cttcctggtc gatcagctcg cgggcgtgcg cgatctgtgc 10500
   cggggtgagg gtagggcggg ggccaaactt cacgcctcgg gccttggcgg cctcgcgccc 10560
   gctccgggtg cggtcgatga ttagggaacg ctcgaactcg gcaatgccgg cgaacacggt 10620
   caacaccatg cggccggccg gcgtggtggt gtcggcccac ggctctgcca ggctacgcag 10680
   gcccgcgccg gcctcctgga tgcgctcggc aatgtccagt aggtcgcggg tgctgcgggc 10740
   caggcggtct agcctggtca ctgtcacaac gtcgccaggg cgtaggtggt caagcatcct 10800
   ggccagctcc gggcggtcgc gcctggtgcc ggtgatcttc tcggaaaaca gcttggtgca 10860
   gccggccgcg tgcagttcgg cccgttggtt ggtcaagtcc tggtcgtcgg tgctgacgcg 10920
   ggcatagccc agcaggccag cggcggcgct cttgttcatg gcgtaatgtc tccggttcta 10980
   gtcgcaagta ttctacttta tgcgactaaa acacgcgaca agaaaacgcc aggaaaaggg 11040
   cagggcggca gcctgtcgcg taacttagga cttgtgcgac atgtcgtttt cagaagacgg 11100
   ctgcactgaa cgtcagaagc cgactgcact atagcagcgg aggggttgga tcgatccctg 11160
   ctcgcgcagg ctgggtgcca agctctcggg taacatcaag gcccgatcct tggagccctt 11220
   gccctcccgc acgatgatcg tgccgtgatc gaaatccaga tccttgaccc gcagttgcaa 11280
   accctcactg atccgcatgc ccgttccata cagaagctgg gcgaacaaac gatgctcgcc 11340
   ttccagaaaa ccgaggatgc gaaccacttc atccggggtc agcaccaccg gcaagcgccc 11400
   ggacggccga ggtcttccga tctcctgaag ccagggcaga tccgtgcaca gcacttgccg 11460
   tagaagaaca gcaaggccgc caatgcctga cgatgcgtgg agaccgaaac cttgcgctcg 11520
   ttcgccagcc aggacagaaa tgcctcgact tcgctgctgc ccaaggttgc cgggtgacgc 11580
   acaccgtgga aacggatgaa ggcacgaacc cagtggacat aagcctgttc ggttcgtaag 11640
   ctgtaatgca agtagcgtat gcgctcacgc aactggtcca gaaccttgac cgaacgcagc 11700
   ggtggtaacg gcgcagtggc ggttttcatg gcttgttatg actgtttttt tggggtacag 11760
   tctatgcctc gggcatccaa gcagcaagcg cgttacgccg tgggtcgatg tttgatgtta 11820
   tggagcagca acgatgttac gcagcagggc agtcgcccta aaacaaagtt aaacatcatg 11880
   agggaagcgg tgatcgccga agtatcgact caactatcag aggtagttgg cgtcatcgag 11940
   cgccatctcg aaccgacgtt gctggccgta catttgtacg gctccgcagt ggatggcggc 12000
   ctgaagccac acagtgatat tgatttgctg gttacggtga ccgtaaggct tgatgaaaca 12060
   acgcggcgag ctttgatcaa cgaccttttg gaaacttcgg cttcccctgg agagagcgag 12120
   attctccgcg ctgtagaagt caccattgtt gtgcacgacg acatcattcc gtggcgttat 12180
   ccagctaagc gcgaactgca atttggagaa tggcagcgca atgacattct tgcaggtatc 12240
   ttcgagccag ccacgatcga cattgatctg gctatcttgc tgacaaaagc aagagaacat 12300
   agcgttgcct tggtaggtcc agcggcggag gaactctttg atccggttcc tgaacaggat 12360
   ctatttgagg cgctaaatga aaccttaacg ctatggaact cgccgcccga ctgggctggc 12420
   gatgagcgaa atgtagtgct tacgttgtcc cgcatttggt acagcgcagt aaccggcaaa 12480
   atcgcgccga aggatgtcgc tgccgactgg gcaatggagc gcctgccggc ccagtatcag 12540
   cccgtcatac ttgaagctag acaggcttat cttggacaag aagaagatcg cttggcctcg 12600
   cgcgcagatc agttggaaga atttgtccac tacgtgaaag gcgagatcac caaggtagtc 12660
   ggcaaataat gtctaacaat tcgttcaagc cgacgccgct tcgcggcgcg gcttaactca 12720
   agcgttagat gcactaagca cataattgct cacagccaaa ctatcaggtc aagtctgctt 12780
   ttattatttt taagcgtgca taataagccc tacacaaatt gggagatata tcatgaaagg 12840
   ctggcttttt cttgttatcg caatagttgg cgaagtaatc gcaacatccg cattaaaatc 12900
   tagcgagggc tttactaagc tagcttgctt ggtcgttccg gtaccgtgaa cgtcggctcg 12960
   attgtacctg cgttcaaata ctttgcgatc gtgttgcgcg cctgcccggt gcgtcggctg 13020
   atctcacgga tcgactgctt ctctcgcaac gccatccgac ggatgatgtt taaaagtccc 13080
   atgtggatca ctccgttgcc ccgtcgctca ccgtgttggg gggaaggtgc acatggctca 13140
   gttctcaatg gaaattatct gcctaaccgg ctcagttctg cgtagaaacc aacatgcaag 13200
   ctccaccggg tgcaaagcgg cagcgg                                      13226

## Description

### Field of the invention.

The present invention relates to the use of poly (ADP-ribose) glycohydrolases in plants to increase the tolerance of plants to adverse growing conditions, including drought, high light intensities, high temperatures, nutrient limitations and the like. Methods and means are provided to produce plants that are tolerant to abiotic stress conditions.

### Background to the invention

. Frequently, abiotic stress will lead either directly or indirectly to damage of the DNA of the cells of the plants exposed to the adverse conditions. Genomic damage, if left unrepaired, can lead to cell death. Tolerance to stress conditions exhibited by plants is the result of the ability of the plant cells exposed to the adverse conditions to reduce and/or repair the damage, and to survive.

Plant cells, like other eukaryotic cells, have evolved an elaborate DNA repair system. The activation of poly(ADP-ribose) polymerase (PARP) by DNA strand breaks is often one of the first cellular responses to DNA damage. PARP catalyzes the post-translational modification of proteins by adding successively molecules of ADP-ribose, obtained from the conversion of nicotineamide dinucleotide (NAD), to form multibranched polymers containing up to 200 ADP-ribose residues (about 40 residues in plants). The dependence of poly(ADP-ribose) synthesis on DNA strand breaks, and the presence of PARP in multiprotein complexes further containing key effectors of DNA repair, replication and transcription reactions, strongly suggests that this posttranslational modification is involved in metabolism of nucleic acids, and DNA repair. There are also indications that poly (ADP -ribose) synthesis is involved in regulation of cell cycle and cell death.

Poly (ADP-ribosylation) of proteins is transient in living cells. The poly (ADP-ribose) polymers are rapidly turned over, being converted to free ADP-ribose by the exoglycosidase and endoglycosidase activity of poly (ADP-ribose) glycohydrolase (PARG; E.C.3.2.1.143). The most proximal unit of ADP ribose on the protein acceptor is hydrolyzed by the action of another enzyme (ADP-ribosyl protein lyase).

In addition to this positive (DNA-repair associated) effect of PARP on cell survival, there is also a negative effect of PARP. The process of activating PARP upon DNA damage is associated with a rapid lowering of NAD+ levels, since each ADP-ribose unit transferred by PARP consumes one molecule of NAD+. NAD+ depletion in turn results in ATP depletion, because NAD+ resynthesis requires at least (depending on the biosynthesis pathway) three molecules of ATP per molecule of NAD+. Furthermore, NAD+ depletion block glyceraldehyde -3-phosphate dehydrogenase activity, which is required to resynthesize ATP during glycolysis. Finally, NAD+ is a key carrier of electrons needed to generate ATP via electron transport and oxidative phosphorylation.

The physiological consequence of NAD+ and ATP depletion has been established in the context of DNA-damage induced cell death. It has been shown that the completion of apoptosis is absolutely dependent on the presence of ATP and that, in the absence of this nucleotide, the type of cellular demise switches from apoptosis to necrosis. Since the cellular lysis associates with necrosis generates further damage to neighboring cells it is preferable for multicellular organisms to favor apoptotic cell death rather than necrosis.

It is thus very important to consider the delicate balance of positive and negative effects of the poly (ADP ribosyl)ation on the potential of a cell to survive DNA damage.

WO 00/04173 describes methods to modulate programmed cell death (PCD) in eukaryotic cells and organisms, particularly plant cells and plants, by introducing of "PCD modulating chimeric genes" influencing the expression and/or apparent activity of endogenous poly-(ADP-ribose) polymerase (PARP) genes. Programmed cell death may be inhibited or provoked. The invention particularly relates to the use of nucleotide sequences encoding proteins with PARP activity for modulating PCD, for enhancing growth rate or for producing stress tolerant cells and organisms.

PARG encoding genes have been identified in a number of animals such as *Rattus norvegicus* (Accession numbers: NM_031339, NW_043030, AB019366, ), *Mus musculus* (Accession numbers: NT_039598, NM_003631, AF079557), *Homo sapiens* (Accession numbers: NT_017696; NM_003631, AF005043), *Bos taurus* (Accession numbers: NM_174138, U78975) *Drosophila melanogaster* (Accession number: AF079556)

In plants, a poly(ADP-ribose) glycohydrolase has been identified by map-based cloning of the wild-type gene inactivated in a mutant affected in clock-controlled transcription of genes in *Arabidopsis* and in photoperiod dependent transition from vegetative growth to flowering (*tej*). The nucleotide sequence of the gene can be obtained from nucleotide databases under the accession number AF394690 (Panda et al., 2002 Dev. Cell. 3, 51-61).

US2002/040490 disclosed 999 isolated nucleotide compositions and sequences for *Arabidopsis thaliana* genes, including PARG, which find use in identifying homologous or related genes; in producing compositions that modulate the expression or function of its encoded protein, mapping functional regions of the protein; and in studying associated physiological pathways.

WO03/000898 describes methods to identify genes, the expression of which are altered in response to pathogen infection, as well as the about 5000 genes and their corresponding promoters identified thereby, of which two sequences (SEQ ID 550 and SEQ ID 3) appear to have high sequence identity with ParG gene sequences.

WO 03/008540 describes a total of 17506 abiotic stress responsive polynucleotides and polypeptides, as well as vectors, expression cassettes, host cells, and plants containing such polynucleotides. Also provided are methods for using such polynucleotides and polypeptides, for example, to alter the responsiveness of a plant to abiotic stress.

### Summary of the invention

The invention provides a method to produce a plant tolerant to stress conditions comprising the steps of providing plant cells with a chimeric gene to create transgenic plant cells, wherein the chimeric gene comprises the following operably linked DNA fragments: a plant-expressible promoter; a DNA region, which when transcribed yields an ParG inhibitory RNA molecule; and a 3' end region involved in transcription termination and polyadenylation. A population of transgenic plant lines is regenerated from the transgenic plant cell; and a stress tolerant plant line is identified within the population of transgenic plant lines. The ParG inhibitory RNA molecule may comprise a nucleotide sequence of at least 20 consecutive nucleotides of the nucleotide sequence of the ParG gene present in the plant cell (the endogenous ParG gene). The ParG inhibitory RNA molecule may also comprise a nucleotide sequence of at least 20 consecutive nucleotides of the complement of the nucleotide sequence of the ParG gene present in the plant cell (the endogenous ParG gene). In yet another embodiment, the parG inhibitory RNA may comprise a sense region comprising a nucleotide sequence of at least 20 consecutive nucleotides of the nucleotide sequence of the ParG gene present in the plant cell and an antisense region comprising a nucleotide sequence of at least 20 consecutive nucleotides of the complement of the nucleotide sequence of the ParG gene present in the plant cell, wherein the sense and antisense region are capable of forming a double stranded RNA region comprising said at least 20 consecutive nucleotides. The chimeric gene may further comprise a DNA region encoding a self-splicing ribozyme between said DNA region coding for parG inhibitory RNA molecule and the 3' end region. Stress conditions may be selected from heat, drought, nutrient depletion, oxidative stress or high light conditions.

In yet another embodiment of the invention, a method is provided to produce a plant tolerant to stress conditions comprising the steps of providing plant cells with a chimeric gene to create transgenic plant cells, comprising a DNA region, which when transcribed yields an ParG inhibitory RNA molecule, whereby the DNA region comprises a nucleotide sequence of at least 21 to 100 nucleotides of a nucleotide sequence encoding a protein comprising the amino acid sequence of SEQ ID No 1, 2 or 16 or at least 21 to 100 nucleotides of a nucleotide sequence of SEQ ID 3, 4, 15 or 23 operably linked to a plant-expressible promoter and a 3' end region involved in transcription termination and polyadenylation; regenerating a population of transgenic plant lines from said transgenic plant cell; and identifying a stress tolerant plant line within the population of transgenic plant lines.

The invention also provides DNA molecules comprising a plant-expressible promoter, operably linked to a DNA region, which when transcribed yields an ParG inhibitory RNA molecule, and to a 3' end region involved in transcription termination and polyadenylation. The ParG inhibitory RNA molecule may comprise a nucleotide sequence of at least 20 consecutive nucleotides of the nucleotide sequence of the ParG gene present in the plant cell (the endogenous ParG gene). The ParG inhibitory RNA molecule may also comprise a nucleotide sequence of at least 20 consecutive nucleotides of the complement of the nucleotide sequence of the ParG gene present in the plant cell (the endogenous ParG gene). In yet another embodiment, the parG inhibitory RNA may comprise a sense region comprising a nucleotide sequence of at least 20 consecutive nucleotides of the nucleotide sequence of the ParG gene present in the plant cell and an antisense region comprising a nucleotide sequence of at least 20 consecutive nucleotides of the complement of the nucleotide sequence of the ParG gene present in the plant cell, wherein the sense and antisense region are capable of forming a double stranded RNA region comprising said at least 20 consecutive nucleotides. The chimeric gene may further comprise a DNA region encoding a self-splicing ribozyme between said DNA region coding for parG inhibitory RNA molecule and the 3' end region. The chimeric gene may also comprise a nucleotide sequence of at least 21 to 100 nucleotides of a nucleotide sequence encoding a protein comprising the amino acid sequence of SEQ ID No 1, 2 or 16 or at least 21 to 100 nucleotides of a nucleotide sequence of SEQ ID 3, 4, 15 or 23.

In yet another embodiment, the invention relates to plant cell comprising the DNA molecule of the invention and plants consisting essentially of such plant cells. Seeds and propagating material of such plants comprising the chimeric genes of the invention are also provided.

The invention also relates to a method for obtaining stress tolerant plants comprising the steps of subjecting a plant cell line or a plant or plant line, to mutagenesis; identifying those plant cells or plants that have a mutation in an endogenous ParG gene resulting in a reduction of the PARG activity; subjecting the identified plant cells or plants to stress conditions and identifying plant cells or plants that tolerate said stress conditions better than control plants. Alternatively, plant cells or plants may be selected for resistance to ParG inhibitors, such as gallotannines, and further treated as described in this paragraph.

### Brief description of the figures.

Figure 1. Schematic representation of the poly-ADP ribose polymeratization / depolymerization cycle by the action of PARP/PARG in a eukaryotic cell.

Figure 2. Diagram of the NAD+ and ATP content of *Arabidopsis* lines under high light stress. Dark boxes represent NAD content under high light conditions expressed as percentage of the value for NAD content determined under low light conditions. Light boxes represent ATP content under high light conditions expressed as percentage of the value for ATP content determined under low light conditions.

Figure 3. Diagram of the NAD+ and ATP content of corn lines under nitrogen depletion stress. Dark boxes represent NAD content while light boxes represent ATP content.

### Detailed description of preferred embodiments

The invention is based, on the one hand, on the demonstration that cells from stress resistant plant lines comprising a chimeric gene reducing the PARP gene expression, exhibited a higher NAD/ATP content under adverse conditions than cells from untransformed plant lines. On the other hand, it has been observed that silencing of the expression of PARG encoding gene in tobacco using a transient silencing RNA vector based on satellite viruses resulted in a similar phenotype as that observed for silencing of PARP encoding gene using the same silencing system. Furthermore, silencing the expression of PARG encoding gene in plants, such as Arabidopsis and tobacco, resulted in plants that were more resistant to stress conditions, such as e.g. those imposed by high light conditions.

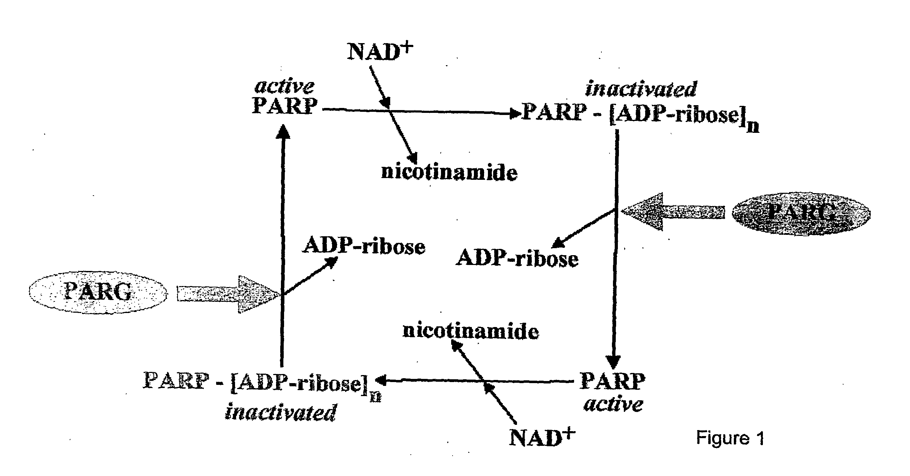

Although not intending to limit the invention to a specific mode of action, it is expected that silencing of PARG gene expression results in a similar phenotype as silencing of PARP gene expression for the following reasons. As can be seen from Figure 1, polymerization of ADP ribose catalyzed by PARP, consuming NAD, is followed by depolymerization of poly ADP ribose, catalyzed by PARG. Poly ADP ribosylation of the PARP protein itself results in inactivation of the PARP protein. The speed at which the ADP ribose polymerization / depolymerization cycle occurs in plant cells, leading to NAD depletion and consequently ATP depletion, can be slowed down or stopped by reduction of the PARP gene expression or of the enzymatic activity of PARP. As a result, plant cells, and plants comprising such cells are more resistant to adverse conditions. The data provided here indicate that a similar effect can be obtained through slowing down or stopping the cycle by reduction of the PARG gene expression or PARG activity.

The invention relates to reduction of plant cell death in response to adverse environmental conditions, and consequently to enhanced stress resistance, by altering the level of expression of ParG genes, or by altering the activity or the apparent activity of PARG proteins in that plant cell. Conveniently, the level of expression of ParG genes may be controlled genetically by introduction of chimeric genes altering the expression of ParG genes, or by altering the endogenous PARG encoding genes, including the expression signals.

In one embodiment of the invention, a method for producing plants tolerant to stress conditions or adverse growing conditions is provided comprising the steps of:
- providing plant cells with a chimeric gene to create transgenic plant cells, wherein the chimeric gene comprises the following operably linked DNA fragments:
   - a plant-expressible promoter;
   - a DNA region, which when transcribed yields a ParG inhibitory RNA molecule;
   - a 3' end region involved in transcription termination and polyadenylation;
- regenerating a population of transgenic plant lines from said transgenic plant cell; and
- identifying a stress tolerant plant line within said population of transgenic plant lines.

As used herein "a stress tolerant plant" or "a plant tolerant to stress conditions or adverse growing conditions" is a plant (particularly a plant obtained according to the methods of the invention), which, when subjected to adverse growing conditions for a period of time, such as but not limited to drought, high temperatures, limited supply of nutrients (particularly nitrogen), high light intensities, grows better than a control plant not treated according to the methods of the invention. This will usually be apparent from the general appearance of the plants and may be measured e.g., by increased biomass production, continued vegetative growth under adverse conditions or higher seed yield. Stress tolerant plant have a broader growth spectrum, i.e. they are able to withstand a broader range of climatological and other abiotic changes, without yield penalty. Biochemically, stress tolerance may be apparent as the higher NAD⁺-NADH /ATP content and lower production of reactive oxygen species of stress tolerant plants compared to control plants under stress condition. Stress tolerance may also be apparent as the higher chlorophyll content, higher photosynthesis and lower chlorophyll fluorescence under stress conditions in stress tolerant plants compared to control plants under the same conditions.

It will be clear that it is also not required that the plant be grown continuously under the adverse conditions for the stress tolerance to become apparent. Usually, the difference in stress tolerance between a plant or plant cell according to the invention and a control plant or plant cell will become apparent even when only a relatively short period of adverse conditions is encountered during growth.

As used herein, a "ParG inhibitory RNA molecule" is an RNA molecule that is capable of decreasing the expression of the endogenous PARG encoding genes of a plant cell, preferably through post-transcriptional silencing. It will be clear that even when a ParG inhibitory RNA molecule decreases the expression of a PARG encoding gene through post-transcriptional silencing, such an RNA molecule may also exert other functions within a cell, such as e.g. guiding DNA methylation of the endogenous ParG gene, again ultimately leading to decreased expression of the PARG encoding gene. Also, expression of the endogenous PARG encoding genes of a plant cell may be reduced by transcriptional silencing, e.g., by using RNAi or dsRNA targeted against the promoter region of the endogenous ParG gene.

As used herein, a "PARG encoding gene" or a "ParG gene" is a gene capable of encoding a PARG (poly ADP ribose glycohydrolase) protein, wherein the PARG protein catalyzes the depolymerization of poly ADP-ribose, by releasing free ADP ribose units either by endoglycolytic or exoglycolytic action.

PARG encoding genes may comprise a nucleotide sequence encoding a protein comprising the amino acid sequence of SEQ ID No 1 (*Arabidopsis thaliana*) or of SEQ ID No 2 (*Solanum tuberosum*) or of SEQ ID No 16 (*Oryza sativa*) or parts thereof, such as a DNA fragment comprising the nucleotide sequence of SEQ ID No. 3 or SEQ ID 4 or SEQ ID No 15. or SEQ ID 23 (Zea *mays*).

However, it will be clear that the skilled person can isolate variant DNA sequences from other plant species, by hybridization with a probe derived from the above mentioned PARG encoding genes from plant species, or even with a probe derived from the above mentioned PARG encoding genes from animal species. To this end, the probes should preferably have a nucleotide sequence comprising at least 40 consecutive nucleotides from the coding region of those mentioned PARG encoding genes sequences, preferably from the coding region of SEQ ID No 3 or SEQ ID No 4. The probes may however comprise longer regions of nucleotide sequences derived from the ParG genes, such as about 50, 60, 75, 100, 200 or 500 consecutive nucleotides from any of the mentioned ParG genes. Preferably, the probe should comprise a nucleotide sequence coding for one of the highly conserved regions of the catalytic domain, which have been identified by aligning the different PARG proteins from animals. These regions are also present in the identified PARG protein from *Arabidopsis thaliana* and comprise the amino acid sequence LXVDFANXXXGGG (corresponding to SEQ ID No 1 from the amino acid at position 252 to the amino acid at position 264; X may be any amino acid) LXVDFANXXXGGGXXXXGXVQEEIRF (corresponding to SEQ ID No 1 from the amino acid at position 252 to the amino acid at position 277) or LXVDFANXXXGGGXXXXGXVQEEIRFXXXPE (corresponding to SEQ ID No 1 from the amino acid at position 252 to the amino acid at position 282), TGXWGCGXFXGD (corresponding to SEQ ID No 1 from the amino acid at position 449 to the amino acid at position 460) or TGXWGCGAFXGDXXLKXXXQ (corresponding to SEQ ID No 1 from the amino acid at position 449 to the amino acid at position 468). Other conserved regions have the amino acid sequence DXXXRXXXXAIDA (corresponding to SEQ ID No 1 from the amino acid at position 335 to the amino acid at position 344) or REXXKAXXGF (corresponding to SEQ ID No 1 from the amino acid at position 360 to the amino acid at position 369) or GXXXXSXYTGY (corresponding to SEQ ID No 1 from the amino acid at position 303 to the amino acid at position 313). Hybridization should preferably be under stringent conditions.

"Stringent hybridization conditions" as used herein mean that hybridization will generally occur if there is at least 95% and preferably at least 97% sequence identity between the probe and the target sequence. Examples of stringent hybridization conditions are overnight incubation in a solution comprising 50% formamide, 5 x SSC (150 mM NaCl, 15 mM trisodium citrate), 50 mM sodium phosphate (pH 7.6), 5x Denhardt's solution, 10% dextran sulfate, and 20 µg/ml denatured, sheared carrier DNA such as salmon sperm DNA, followed by washing the hybridization support in 0.1 x SSC at approximately 65°C, e.g. for about 10 min (twice). Other hybridization and wash conditions are well known and are exemplified in Sambrook et al, Molecular Cloning: A Laboratory Manual, Second Edition, Cold Spring Harbor, NY (1989), particularly chapter 11.

Alternatively, ParG encoding genes or parts thereof may also be isolated by PCR based techniques, using as primers oligonucleotides comprising at least 20 consecutive nucleotides from a nucleotide sequence of the mentioned PARG encoding genes or the complement thereof. Such primers may comprise a nucleotide sequence encoding a conserved region, as mentioned above, or be complementary to such a nucleotide sequence. Oligonucleotides which may be used for that purpose may comprise the nucleotide sequence of either or SEQ ID No.5, SEQ ID No 6., SEQ ID No. 7 or SEQ ID No. 8. Oligonucleotides which may be used may also be degenerate, such as the oligonucleotide primers of SEQ ID No 17, SEQ ID No 18, SEQ ID No 19; SEQ ID No 20, SEQ ID No 21 or SEQ ID No 22.

Specific PCR fragments from ParG genes may e.g., be obtained by using combinations of the oligonucleotides having the nucleotide sequence of SEQ ID No. 5 and SEQ ID No 6 using e.g., *Arabidopsis* genomic DNA or cDNA as a template DNA, or by using combinations of the oligonucleotides having the nucleotide sequence of SEQ ID No. 7 and SEQ ID No 8 using e.g., potato genomic DNA or cDNA as a template DNA, under stringent annealing conditions.

The isolated sequences may encode a functional PARG protein or a part thereof. Preferably the isolated sequences should comprise a nucleotide sequence coding for one or more of the highly conserved regions from the catalytic domain of PARG proteins as mentioned elsewhere.

However, for the purpose of the invention is not required that the isolated sequences encode a functional ParG protein nor that a complete coding region is isolated. Indeed, all that is required for the invention is that a chimeric gene can be designed or produced, based on the identified or isolated sequence of the endogenous ParG gene from a plant, which is capable of producing a ParG inhibitory RNA. Several alternative methods are available to produce such a ParG inhibitory RNA molecule.

In one embodiment, the ParG inhibitory RNA molecule encoding chimeric gene is based on the so-called antisense technology. In other words, the coding region of the chimeric gene comprises a nucleotide sequence of at least 20 consecutive nucleotides of the complement of the nucleotide sequence of the endogenous ParG gene of the plant cell or plant, the expression of which is targeted to be reduced. Such a chimeric gene may be conveniently constructed by operably linking a DNA fragment comprising at least 20 nucleotides from the isolated or identified ParG gene, or part of such a gene, in inverse orientation, to a plant expressible promoter and 3'end formation region involved in transcription termination and polyadenylation. It will be immediately clear that there is no need to know the exact nucleotide sequence or complete nucleotide sequence of such a DNA fragment from an isolated ParG gene.

In another embodiment the ParG inhibitory RNA molecule encoding chimeric gene is based on the so-called co-suppression technology. In other words, the coding region of the chimeric gene comprises a nucleotide sequence of at least 20 consecutive nucleotides of the nucleotide sequence of the endogenous ParG gene of the plant cell or plant, the expression of which is targeted to be reduced. Such a chimeric gene may be conveniently constructed by operably linking a DNA fragment comprising at least 20 nucleotides from the isolated or identified ParG gene, or part of such a gene, in direct orientation, to a plant expressible promoter and 3'end formation region involved in transcription termination and polyadenylation. Again it is not required to know the exact nucleotide sequence of the used DNA fragment from the isolated ParG gene.

The efficiency of the above mentioned chimeric genes in reducing the expression of the endogenous ParG gene may be further enhanced by inclusion of DNA elements which result in the expression of aberrant, unpolyadenylated ParG inhibitory RNA molecules. One such DNA element suitable for that purpose is a DNA region encoding a self-splicing ribozyme, as described in WO 00/01133.

The efficiency or the above mentioned chimeric genes in reducing the expression of the endogenous ParG gene of a plant cell may also be further enhanced by including into one plant cell simultaneously a chimeric gene as herein described encoding a antisense ParG inhibitory RNA molecule and a chimeric gene as herein described encoding a sense ParG inhibitory RNA molecule, wherein said antisense and sense ParG inhibitory RNA molecules are capable of forming a double stranded RNA region by base pairing between the mentioned at least 20 consecutive nucleotides, as described in WO 99/53050.

As further described in WO 99/53050, the sense and antisense ParG inhibitory RNA regions, capable of forming a double stranded RNA region may be present in one RNA molecule, preferably separated by a spacer region. The spacer region may comprise an intron sequence. Such a chimeric gene may be conveniently constructed by operably linking a DNA fragment comprising at least 20 nucleotides from the isolated or identified endogenous ParG gene, the expression of which is targeted to be reduced, in an inverted repeat, to a plant expressible promoter and 3'end formation region involved in transcription termination and polyadenylation. To achieve the construction of such a chimeric gene, use can be made of the vectors described in WO 02/059294

An embodiment of the invention thus concerns a method for obtaining a stress tolerant plant line comprising the steps of
- providing plant cells with a chimeric gene to create transgenic plant cells, wherein the chimeric gene comprises the following operably linked DNA fragments:
   - a plant-expressible promoter;
   - a DNA region, which when transcribed yields a ParG inhibitory RNA molecule comprising a nucleotide sequence of at least 20 consecutive nucleotides of the nucleotide sequence of the ParG gene present in said plant cell; or
   - a DNA region, which when transcribed yields a ParG inhibitory RNA molecule comprising a nucleotide sequence of at least 20 consecutive nucleotides of the complement of the nucleotide sequence of the ParG gene present in said plant cell; or
   - a DNA region, which when transcribed yields a ParG inhibitory RNA molecule comprising a sense region comprising a nucleotide sequence of at least 20 consecutive nucleotides of the nucleotide sequence of the ParG gene present in said plant cell and an antisense region comprising a nucleotide sequence of at least 20 consecutive nucleotides of the complement of the nucleotide sequence of the ParG gene present in said plant cell, wherein said sense and antisense region are capable of forming a double stranded RNA region comprising said at least 20 consecutive nucleotides.
   - a 3' end region involved in transcription termination and polyadenylation;
- regenerating a population of transgenic plant lines from said transgenic plant cell; and
- identifying a stress tolerant plant line within said population of transgenic plant lines.

As used herein "comprising" is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, i.e., be embedded in a larger nucleic acid or protein. A chimeric gene comprising a DNA region which is functionally or structurally defined, may comprise additional DNA regions etc.

It will thus be clear that the minimum nucleotide sequence of the antisense or sense RNA region of about 20 nt of the ParG coding region may be comprised within a larger RNA molecule, varying in size from 20 nt to a length equal to the size of the target gene.

The mentioned antisense or sense nucleotide regions may thus be about from about 21 nt to about 5000 nt long, such as 21 nt, 40 nt, 50 nt, 100nt, 200 nt, 300nt, 500nt, 1000 nt, 2000 nt or even about 5000 nt or larger in length.

Moreover, it is not required for the purpose of the invention that the nucleotide sequence of the used inhibitory ParG RNA molecule or the encoding region of the chimeric gene, is completely identical or complementary to the endogenous ParG gene the expression of which is targeted to be reduced in the plant cell. The longer the sequence, the less stringent the requirement for the overall sequence identity is. Thus, the sense or antisense regions may have an overall sequence identity of about 40 % or 50% or 60 % or 70% or 80% or 90 % or 100% to the nucleotide sequence of the endogenous ParG gene or the complement thereof. However, as mentioned antisense or sense regions should comprise a nucleotide sequence of 20 consecutive nucleotides having about 100% sequence identity to the nucleotide sequence of the endogenous ParG gene. Preferably the stretch of about 100 % sequence identity should be about 50, 75 or 100 nt.

For the purpose of this invention, the "sequence identity" of two related nucleotide sequences, expressed as a percentage, refers to the number of positions in the two optimally aligned sequences which have identical residues (x100) divided by the number of positions compared. A gap, i.e. a position in an alignment where a residue is present in one sequence but not in the other is regarded as a position with non-identical residues. The alignment of the two sequences is performed by the Needleman and Wunsch algorithm (Needleman and Wunsch 1970) Computer-assisted sequence alignment, can be conveniently performed using standard software program such as GAP which is part of the Wisconsin Package Version 10.1 (Genetics Computer Group, Madison, Wisconsin, USA) using the default scoring matrix with a gap creation penalty of 50 and a gap extension penalty of 3.

It will be clear that whenever nucleotide sequences of RNA molecules are defined by reference to nucleotide sequence of corresponding DNA molecules, the thymine (T) in the nucleotide sequence should be replaced by uracil (U). Whether reference is made to RNA or DNA molecules will be clear from the context of the application.

It will also be clear that chimeric genes capable of producing inhibitory ParG genes for a particular ParG gene in a particular plant variety or plant species, may also be used to inhibit ParG gene expression in other plant varieties or plant species. Indeed, when sufficient homology exists between the ParG inhibitory RNA region and the ParG gene, or when the ParG genes share the same stretch of 19 nucleotides, expression of those other genes will also be down-regulated.

In view of the potential role of ParG in nucleic acid metabolism, it may be advantageous that the expression of the endogenous ParG gene by the ParG inhibitory RNA is not completely inhibited. Downregulating the expression of a particular gene by gene silencing through the introduction of a chimeric gene encoding ParG inhibitory RNA will result in a population of different transgenic lines, exhibiting a distribution of different degrees of silencing of the ParG gene. The population will thus contain individual transgenic plant lines, wherein the endogenous ParG gene is silenced to the required degree of silencing. A person skilled in the art can easily identify such plant lines, e.g. by subjecting the plant lines to a particular adverse condition, such a high light intensity, oxidative stress, drought, heat etc. and selecting those plants which perform satisfactory and survive best the treatment.

As used herein, the term "promoter" denotes any DNA which is recognized and bound (directly or indirectly) by a DNA-dependent RNA-polymerase during initiation of transcription. A promoter includes the transcription initiation site, and binding sites for transcription initiation factors and RNA polymerase, and can comprise various other sites (e.g., enhancers), at which gene expression regulatory proteins may bind.

The term "regulatory region", as used herein, means any DNA, that is involved in driving transcription and controlling (i.e., regulating) the timing and level of transcription of a given DNA sequence, such as a DNA coding for a protein or polypeptide. For example, a 5' regulatory region (or "promoter region") is a DNA sequence located upstream (i.e., 5') of a coding sequence and which comprises the promoter and the 5'-untranslated leader sequence. A 3' regulatory region is a DNA sequence located downstream (i.e., 3') of the coding sequence and which comprises suitable transcription termination (and/or regulation) signals, including one or more polyadenylation signals.

In one embodiment of the invention the promoter is a constitutive promoter. In another embodiment of the invention, the promoter activity is enhanced by external or internal stimuli (inducible promoter), such as but not limited to hormones, chemical compounds, mechanical impulses, abiotic or biotic stress conditions. The activity of the promoter may also regulated in a temporal or spatial manner (tissue-specific promoters; developmentally regulated promoters).

For the purpose of the invention, the promoter is a plant-expressible promoter. As used herein, the term "plant-expressible promoter" means a DNA sequence which is capable of controlling (initiating) transcription in a plant cell. This includes any promoter of plant origin, but also any promoter of non-plant origin which is capable of directing transcription in a plant cell, i.e., certain promoters of viral or bacterial origin such as the CaMV35S (Hapster et al., 1988), the subterranean clover virus promoter No 4 or No 7 (WO9606932), or T-DNA gene promoters but also tissue-specific or organ-specific promoters including but not limited to seed-specific promoters (e.g., WO89/03887), organ-primordia specific promoters (An et al., 1996), stem-specific promoters (Keller et al., 1988), leaf specific promoters (Hudspeth et al., 1989), mesophyl-specific promoters (such as the light-inducible Rubisco promoters), root-specific promoters (Keller et al.,1989), tuber-specific promoters (Keil et al., 1989), vascular tissue specific promoters ( Peleman et al., 1989 ), stamen-selective promoters ( WO 89/10396, WO 92/13956), dehiscence zone specific promoters (WO 97/13865) and the like.

Methods for the introduction of chimeric genes into plants are well known in the art and include *Agrobacterium*-mediated transformation, particle gun delivery, microinjection, electroporation of intact cells, polyethyleneglycol-mediated protoplast transformation, electroporation of protoplasts, liposome-mediated transformation, silicon-whiskers mediated transformation etc. The transformed cells obtained in this way may then be regenerated into mature fertile plants.

The transgenic plant cells and plant lines according to the invention may further comprise chimeric genes which will reduce the expression of PARP genes as described in WO 00/04173. These further chimeric genes may be introduced e.g. by crossing the transgenic plant lines of the current invention with transgenic plants containing PARP gene expression reducing chimeric genes. Transgenic plant cells or plant lines may also be obtained by introducing or transforming the chimeric genes of the invention into transgenic plant cells comprising the PARP gene expression reducing chimeric genes or vice versa. Alternatively, the PARP and PARG inhibitory RNA regions may be encoded by one chimeric gene and transcribed as one RNA molecule.

The chimeric genes of the invention (or the inhibitory RNA molecules corresponding thereto) may also be introduced into plant cells in a transient manner, e.g using the viral vectors, such as viral RNA vectors as described in WO 00/63397 or WO 02/13964.

Having read this specification, it will be immediately clear to the skilled artisan, that mutant plant cells and plant lines, wherein the PARG activity is reduced may be used to the same effect as the transgenic plant cells and plant lines described herein. Mutants in ParG gene of a plant cell or plant may be easily identified using screening methods known in the art, whereby chemical mutagenesis, such as e.g., EMS mutagenesis, is combined with sensitive detection methods (such as e.g., denaturing HPLC). An example of such a technique is the so-called "Targeted Induced Local Lesions in Genomes" method as described in McCallum et al, Plant Physiology 123 439-442 or WO 01/75167. However, other methods to detect mutations in particular genome regions or even alleles, are also available and include screening of libraries of existing or newly generated insertion mutant plant lines, whereby pools of genomic DNA of these mutant plant lines are subjected to PCR amplification using primers specific for the inserted DNA fragment and primers specific for the genomic region or allele, wherein the insertion is expected ( see e.g. Maes et al., 1999, Trends in Plant Science, 4, pp 90-96).

Plant cell lines and plant lines may also be subjected to mutagenesis by selection for resistance to ParG inhibitors, such as gallotannines . (Ying, et al. (2001). Proc. Natl. Acad. Sci. USA 98(21), 12227-12232; Ying, W., Swanson, R.A. (2000). NeuroReport 11 (7), 1385-1388.

Thus, methods are available in the art to identify plant cells and plant lines comprising a mutation in the ParG gene. This population of mutant cells or plant lines can then be subjected to different abiotic stresses, and their phenotype or survival can be easily determined. Additionally, the NAD and/or the ATP content of the stressed cells can be determined and compared to results of such determinations of unstressed cells. In stress tolerant cells, the reduction of NAD content under stress conditions should when compared with unstressed cells, should be lower than for corresponding control cells.

It is also an object of the invention to provide plant cells and plants containing the chimeric genes or the RNA molecules according to the invention. Gametes, seeds, embryos, either zygotic or somatic, progeny or hybrids of plants comprising the chimeric genes of the present invention, which are produced by traditional breeding methods are also included within the scope of the present invention.

The plants obtained by the methods described herein may be further crossed by traditional breeding techniques with other plants to obtain stress tolerant progeny plants comprising the chimeric genes of the present invention.

The methods and means described herein are believed to be suitable for all plant cells and plants, both dicotyledonous and monocotyledonous plant cells and plants including but not limited to cotton, Brassica vegetables, oilseed rape, wheat, corn or maize, barley, alfalfa, peanuts, sunflowers, rice, oats, sugarcane, soybean, turf grasses, barley, rye, sorghum, sugar cane, vegetables (including chicory, lettuce, tomato, zucchini, bell pepper, eggplant, cucumber, melon, onion, leek), tobacco, potato, sugarbeet, papaya, pineapple, mango, *Arabidopsis thaliana,* but also plants used in horticulture, floriculture or forestry (poplar, fir, eucalyptus etc.).

The following non-limiting Examples describe method and means for increasing stress tolerance in plants according to the invention.

Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK. Other references for standard molecular biology techniques include Sambrook and Russell (2001) Molecular Cloning: A Laboratory Manual, Third Edition, Cold Spring Harbor Laboratory Press, NY, Volumes I and II of Brown (1998) Molecular Biology LabFax, Second Edition, Academic Press (UK). Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

Throughout the description and Examples, reference is made to the following sequences:

SEQ ID N°1: amino acid sequence of the ParG protein from *Arabidopsis thaliana.*

SEQ ID N°2: amino acid sequence of part of the ParG protein from *Solanum tuberosum.*

SEQ ID N°3: nucleotide sequence encoding the ParG protein from *Arabidopsis thaliana.*

SEQ ID N°4: nucleotide sequence encoding the part of the ParG protein from *Solanum tuberosum.*

SEQ ID N°5: nucleotide sequence of an oligonucleotide primer suitable for PCR amplification of part of a ParG protein encoding DNA fragment.

SEQ ID N°6: nucleotide sequence of an oligonucleotide primer suitable for PCR amplification of part of a ParG protein encoding DNA fragment.

SEQ ID N°7: nucleotide sequence of an oligonucleotide primer suitable for PCR amplification of part of a ParG protein encoding DNA fragment.

SEQ ID N°8: nucleotide sequence of an oligonucleotide primer suitable for PCR amplification of part of a ParG protein encoding DNA fragment.

SEQ ID N°9: nucleotide sequence of the T-DNA vector containing the ParG expression reducing chimeric gene based on the *Arabidopsis* ParG gene sequence.

SEQ ID N°10: amino acid sequence of conserved sequence 1 of PARG proteins.

SEQ ID N°11: amino acid sequence of conserved sequence 2 of PARG proteins.

SEQ ID N°12: amino acid sequence of conserved sequence 3 of PARG proteins.

SEQ ID N°13: amino acid sequence of conserved sequence 4 of PARG proteins.

SEQ ID N°14: amino acid sequence of conserved sequence 5 of PARG proteins.

SEQ ID N°15: nucleotide sequence of the ParG protein from *Oryza sativa.*

SEQ ID N°16: amino acid sequence of the ParG protein from *Oryza sativa.*

SEQ ID N° 17: nucleotide sequence of an oligonucleotide primer PG1 suitable for PCR amplification of part of a ParG protein encoding DNA fragment.

SEQ ID N° 18: nucleotide sequence of an oligonucleotide primer PG2 suitable for PCR amplification of part of a ParG protein encoding DNA fragment.

SEQ ID N° 19: nucleotide sequence of an oligonucleotide primer PG3 suitable for PCR amplification of part of a ParG protein encoding DNA fragment.

SEQ ID N° 20: nucleotide sequence of an oligonucleotide primer PG4 suitable for PCR amplification of part of a ParG protein encoding DNA fragment.

SEQ ID N° 21: nucleotide sequence of an oligonucleotide primer PG5 suitable for PCR amplification of part of a ParG protein encoding DNA fragment.

SEQ ID N° 22: nucleotide sequence of an oligonucleotide primer PG6 suitable for PCR amplification of part of a ParG protein encoding DNA fragment.

SEQ ID N°23: nucleotide sequence encoding a ParG protein from Zea *mays.*

SEQ ID N°24: nucleotide sequence of a T-DNA vector comprising a chimeric gene capable of reducing PARG expression

SEQ ID N°25: nucleotide sequence of a T-DNA vector comprising a chimeric gene capable of reducing PARG expression

**Examples**

### Example 1. Analysis of the influence of stress on energy production efficiency of transgenic stress tolerant plant lines containing PARP gene expresssion reducing chimeric genes.

Hypocotyls of transgenic *Brassica napus* plants comprising PARP gene expression reducing chimeric genes as described in WO 00/04173 were cultivated for 5 days on a growth medium. Explants were then transferred to liquid medium comprising 30 mg/L aspirin or acetylsalicylic acid (resulting in oxidative stress conditions) for one day. In control experiments, hypocotyls of non-transgenic *Brassica napus* plants N90-740 were cultivated on the same growth medium and then incubated for one day in liquid medium comprising 30 mg/L aspirin. In addition, hypocotyls of both the transgenic lines and the control line were cultivated on the same growth medium without aspirin.

After the cultivation period, the ATP content of 125 explants was determined for each experiment. Additionally, the oxygen consumed in 3 hours by 125 explants was determined. The results are summarized in Table 1. The standard error of the mean was less than 6%. Whereas, the ratio of moles ATP per mg consumed oxygen in the control plants decreased in the control plants when oxidative stress was applied, the same ratio in the stress tolerant transgenic plant lines actually increased under stress conditions, and was considerably higher (about 24%) than in the control plants. The stress resistant transgenic lines thus maintained an constant energy production efficiency, whereas the control lines exhibited an decreased energy production efficiency. In addition, superoxide production, expressed as a percentage of superoxide production in control plants not subjected to the oxidative stress, did not increase in stress tolerant plants subjected to stress conditions.

**Table 1. Influence of stress on energy production efficiency of 5 days cultured Brassica napus hypocotyl explants.**

| **Plant line** | **Stress** | **moles ATP per 125 explants** | **O₂ mg/L consumed in 3 hrs by 125 explants** | **moles ATP** | **Superoxide production** |
|---|---|---|---|---|---|
| | | | | **mg consumed O₂** | |
| N90-740 (control) | None | 12.4 x 10⁻⁷ | 2.96 | 4.19 x 10⁻⁷ | 100% |
| | 30mg/L aspirin | 13.2 x 10⁻⁷ | 4.06 | 3.25 x 10⁻⁷ | 167% |
| Transgenic line | None | 9.3 x 10⁻⁷ | 2.33 | 3.99 x 10⁻⁷ | 108% |
| | 30mg/L aspirin | 11.4 x 10⁻⁷ | 2.82 | 4.04 x 10⁻⁷ | 100% |

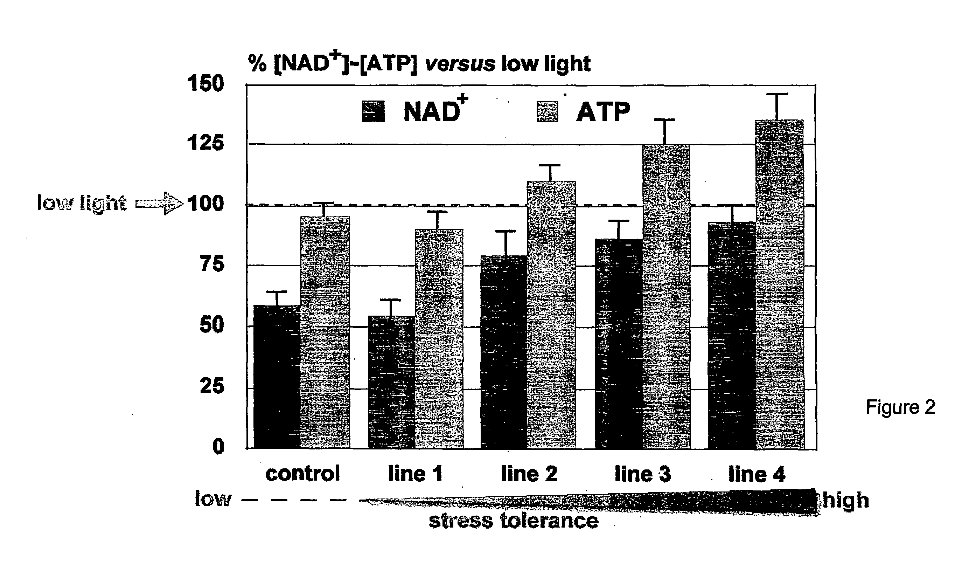

In another experiment, the NAD+ and ATP content of 4 different transgenic *Arabidopsis* lines comprising PARP gene expression reducing chimeric genes as described in WO 00/04173 were determined under high and low light conditions, and compared to the values obtained for a non transformed control line under the same conditions. The 4 different lines exhibited different degrees of stress resistance as exhibited e.g. by their ability to withstand heat and/or drought conditions. The values obtained for the NAD and ATP contents under high light stress are expressed as a percentage of the values for the NAD and ATP contents under low light conditions, and are plotted in Figure 2.

The results show that high light stress leads to a significant NAD reduction in control plant cells and in the transgenic plant line which is the least stress resistant. The more stress resistant the transgenic plant lines are, the less signicifant the NAD reduction is under high light stress conditions.

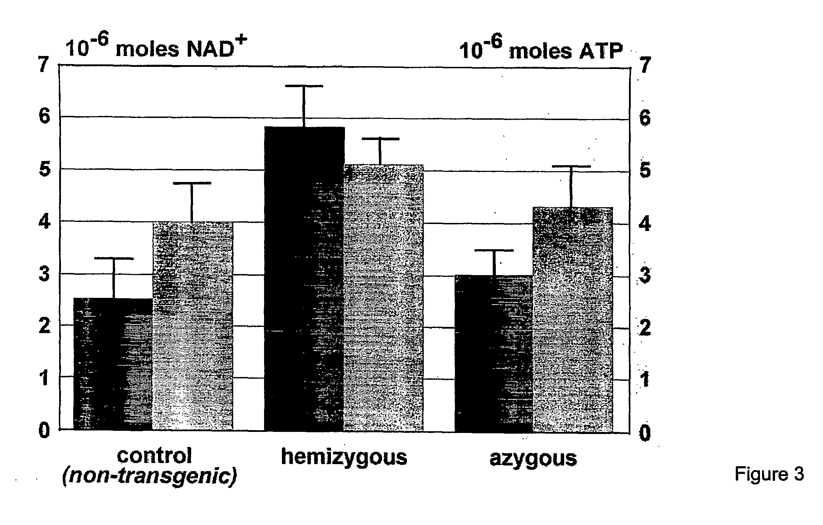

In another experiment, the NAD+ and ATP content of a segregating population resulting from a cross between transgenic corn lines comprising PARP gene expression reducing chimeric genes as described in WO 00/04173 and an untransformed corn line, were determined under conditions of nutrient (nitrogen) depletion, and compared to the values obtained for a non transformed control line under the same conditions. Figure 3 is a graphic representation of the of the obtained results. Hemizygous and azygous lines were discriminated by verification for the presence of the selectable marker gene. The NAD and ATP content was significantly higher in the hemizygous, stress tolerant plants than in the untransformed control plants or the azygous plants.

### Example 2. Construction of ParG gene expression reducing chimeric genes.

To reduce the expression of the PARG gene e.g. in *Arabidopsis* and related plants, a chimeric gene was constructed which is capable expressing a dsRNA comprising both a sense and antisense region which can form a double stranded RNA. Such dsRNA is very effective in reducing the expression of the genes with which is shares sequence homology, by post-transcriptional silencing. The chimeric gene comprises the following DNA fragments:
- A promoter region from Cauliflower mosaic Virus (CaMV 35S);
- A DNA fragment comprising 163 bp from the ParG gene from *Arabidopsis thaliana* in direct orientation (Genbank Accession number AF394690 from nucleotide position 973 to 1135);
- A DNA fragment encoding intron 2 from the pdk gene from Flaveria;
- The DNA fragment comprising 163 bp from the ParG gene from *Arabidopsis thaliana* in inverted orientation (Genbank Accession number AF394690 from nucleotide position 973 to 1135)
- A fragment of the 3' untranslated end from the octopine synthetase gene from *Agrobacterium tumefaciens.*

This chimeric gene was introduced in a T-DNA vector, between the left and right border sequences from the T-DNA, together with a selectable marker gene providing resistance to the herbicide phosphinotricin.

To reduce the expression of the PARG gene e.g. in potatoes and related plants, a chimeric gene is constructed which is capable expressing a dsRNA comprising both a sense and antisense region of a cDNA sequence from potato, that is capable of encoding a protein having high sequence identity with the N-terminal part of the *Arabidopsis* PARG protein. The chimeric gene comprises the following DNA fragments:
- A promoter region from Cauliflower mosaic Virus (CaMV 35S);
- A DNA fragment comprising a sequence of at least 100 bp from ParG homologue from *Solanum tuberosum* in direct orientation (Genbank Accession number BE340510);
- A DNA fragment encoding intron 2 from the pdk gene from Flaveria;
- The DNA fragment comprising the sequence of at least 100 bp from ParG homologue from *Solanum tuberosum* in inverted orientation (Genbank Accession number BE340510);
- A fragment of the 3' untranslated end from the octopine synthetase gene from *Agrobacterium tumefaciens*

This chimeric gene is introduced in a T-DNA vector, between the left and right border sequences from the T-DNA, together with a selectable marker gene providing resistance to the herbicide phosphinotricin.

### Example 3. Analysis of transgenic plant lines comprising ParG gene expression reducing chimeric genes.

The chimeric genes of Example 2 are introduced into *Arabidopsis* or potato respectively, by Agrobacterium mediated transformation.

The population of obtained transgenic lines is subjected to the following stress conditions, together with control plants:
- Increased heat for a period of days (greenhouse) or hours (in vitro)
- Drought for a period of days
- High light conditions for a period of days
- Nutrient depletion

Individual plant lines surviving well the above mendioned stress conditions are selected.

The NAD content and ATP content for the above mentioned plants is determined under control and stress conditions.

### Example 4. Quantitative determination of NAD, ATP and superoxide radicals in plant cells.

Quantification of ATP in plant tissues was done basically as decribed by Rawyler et al. (1999), Plant Physiol. 120, 293-300. The assay was used for the determination of the ATP content of hypocotyl explants that were cultured for 4-5 days on A2S3 medium or 2 weeks old *in vitro* cultured *Arabidopsis* plants. All manipulations are performed on crushed ice unless otherwhise indicated.

ATP extraction
- Freeze plant material with liquid nitrogen
   ■ 100 hypocotyl explants
   ■ ± 700mg *Arabidopsis* plants (roots + shoots) (about 32-37 18-days old C24 plants)
- Put frozen hypocotyls in mortar and add 6ml of 6% perchloric acid.
- Extraction can be done at room temperature using a pestle. After extraction, put samples as soon as possible on ice.
- Centrifuge at 24,000g (Sorvall, SS34 rotor at 14,000rpm) for 10min. at 4°C.
- The supernatant is neutralized with 5M K₂CO₃ (add 350µl of 5M K₂CO₃ to 3ml of supernatant).
- KClO₄ is removed by spinning as described above.

Quantitative bioluminescent determination of ATP
- The ATP bioluminescent assay kit from Sigma is used (FL-AA).
- Dilute extract 6000 x (about 6 mL extract from which 100µl is taken, that is diluted 1000 times) The dilutions are made with the 'ATP assay mix dilution buffer' (FL-AAB) of the ATP bioluminescent assay kit
- The amount of light that is produced is measured with the TD-20/20 luminometer of Turner Designs (Sunnyvale, USA).
- Standard curve: disolve ATP standard of kit (FL-AAS) in 10ml of water (2x10⁻⁶ moles)

Quantification of NAD+ and NADH in plant tissues was performed, essentially as described by Karp et al. (1983) or Filipovic et al. (1999) on the following plant material:
Brassica napus: 150 5-days cultured hypocotyl explants/sample *Arabidopsis*: 1000mg 18-days old in vitro grown plants (shoots + roots)/sample (corresponds to ±60 C24 plants)

Assay solution
(A) For measuring NADH: 25mM potassium phosphate buffer pH7
   0.1mM DTT
   3µM FMN (Fluka, 83810)
   30µM n-decanal (Sigma, D-7384)
(B) For measuring NAD⁺ + NADH:
   idem as for measuring NADH alone + 2µg/mL alcohol dehydrogenase (Roche, 102 717)

Extraction
- Freeze with liquid nitrogen
- Put frozen plant material in cooled mortar (cooled at -20°C) and add 5mL extraction buffer
- Grind material using a pestle
- Centrifuge at 24 000g (Sorvall, SS34 rotor at 14 000rpm) for 15 minutes at 4°C
- Take 1 mL of supernatant for analysis

Assay
***NADH***
- 390µL of assay solution A
- + 10µL extract
- + 2µL NAD(P)H:FMN oxidoreductase
- + 100µL luciferase solution
***NAD*⁺+ *NADH***
- 390µL of assay solution B
- + 10µL extract
- 2 minutes at room temperature
- + 2µL NAD(P)H:FMN oxidoreductase
- + 100µL luciferase solution
The amount of light that is produced is measured with the TD-20/20 luminometer of Turner Designs (Sunnyvale, USA)
***NADH-standard***
NADH stock solution: 1 mM (7.1mg/10mL H₂O) NADH: disodium salt, Roche, 107 735
Dilution series in 10mM potassium phosphate buffer pH7: (10⁻²); 5x10⁻³; 2x10⁻³; 10⁻³; 5x10⁻⁴
Add 10µL of dilutions in 390µL of assay solution A and perform reaction Make standard curve

Superoxide radicals production was measured by quantifying the reduction of XTT as described in De Block and De Brouwer (2002) Plant Physiol. Biochem. 40, 845-852

BRASSICA NAPUS

Media and reaction buffers
Sowing medium (medium 201):
Half concentrated Murashige and Skoog salts
2% sucrose
pH 5.8
0.6% agar (Difco Bacto Agar)
250mg/l triacillin
Callus inducing medium A2S3:
MS medium, 0.5g/l Mes (pH 5.8), 3% sucrose, 40mg/l adenine-SO₄,
0.5% agarose, 1mg/l 2,4-D, 0.25mg/l NAA, 1mg/l BAP, 250mg/l triacillin
Incubation medium:
25mM K-phosphate buffer pH5.8
2% sucrose
1 drop Tween20 for 25ml medium
Reaction buffer:
50mM K-phosphate buffer pH7.4
1 mM sodium,3'-{1-[phenylamino-carbonyl]-3,4-tetrazolium}-bis(4-methoxy-6-nitro) = XTT (bts, Germany, cat n° 2525)
1 drop Tween20 for 25ml buffer

Sterilization of seeds - pregermination of seeds - growing of the seedlings. Seeds are soaked in 70% ethanol for 2 min, then surface-sterilized for 15 min in a sodium hypochlorite solution (with about 6% active chlorine) containing 0.1% Tween20. Finally, the seeds are rinsed with 1l of sterile tap water. Incubate seeds for at least one hour in sterile tap water (to allow diffusion from seeds of components that may inhibit germination). Seeds are put in 250ml erlenmeyer flasks containing 50ml of sterile tap water (+ 250mg/l triacillin). Shake for about 20 hours. Seeds from which the radicle is protruded are put in Vitro Vent containers from Duchefa containing about 125ml of sowing medium (10 seeds/vessel, not too many to reduce loss of seed by contamination). The seeds are germinated at ±24°C and 10-30:Einstein/s⁻¹m⁻² with a daylength of 16h.

Preculture of the hypocotyl explants and induction of stress
- 12-14 days after sowing, the hypocotyls are cut in about 7-10mm segments.
- The hypocotyl explants (25 hypocotyls/Optilux Petridish, Falcon S1005, Denmark) are cultured for 5 days on medium A2S3 at 25°C (at 10-30□Einstein/s⁻¹m⁻²).

XTT-assay
- Transfer 150 hypocotyl explants to a 50ml Falcon tube.
- Wash with reaction buffer (without XTT).
- Add 20mL reaction buffer + XTT.
   (explants have to be submerged, but do not vacuum infiltrate)
- Incubate in the dark at 26°C for about 3hours
- Measure the absorption of the reaction medium at 470nm

*ARABIDOPSIS THALIANA*

Media and reaction buffers
Plant medium:
Half concentrated Murashige and Skoog salts
B5 vitamins
1.5% sucrose
pH 5.8
0.7% Difco agar
Incubation medium:
10mM K-phosphate buffer pH5.8
2% sucrose
1 drop Tween20 for 25ml medium
Reaction buffer:
50mM K-phosphate buffer pH7.4
1 mM sodium,3'-{1-[phenylamino-carbonyl]-3,4-tetrazolium}-bis(4-methoxy-6-nitro) = XTT (bts, Germany, cat n° 2525) 1 drop Tween20 for 25ml buffer

*Arabidopsis* plants
- *Arabidopsis* lines: control
   lines to test
- Sterilization of *Arabidopsis* seeds:
   2min. 70% ethanol
   10 min. bleach (6% active chlorine) + 1drop Tween 20 for 20ml solution
   wash 5 times with sterile tap water
- Pregermination of seeds:
   *In 9cm Optilux Petridishes (Falcon) containing 12ml sterile tap water.* Low light overnight to 24 hours.
- Growing of *Arabidopsis* plants
   Seeds are sown in Intergrid Tissue Culture disks of Falcon (nr. 3025) containing ±125ml of plant medium: 1 seed/grid.
   Plants are grown at 24°C
   30µEinstein s⁻¹m⁻²
   16hours light - 8hours dark
   for about 3 weeks (before bolting)

XTT-assay
*Control condition (no stress)*
   - Harvest shoots (roots included) from agar plates and put them directly in a 50ml Falcon tube containing reaction buffer (without XTT)
*Stressed shoots*
   - Transfer shoots to 50ml Falcon tubes containing reaction buffer (without XTT)
   - Replace reaction buffer with buffer containing XTT (40mL/tube)
   - Shoots have to be submerged, but do not vacuum infiltrate
   - Incubate in the dark at 26°C for about 3hours
   - Measure the absorption of the reaction medium at 470nm

Quantification of respiration by measuring oxygen consumption using a Clark polarographic electrode was done in the following way:

Plant material
*Brassica napus*
   150-200* hypocotyl explants
   Cultured for 5 days at 25°C
   (*cfr*. protocol vigour assay)
      * 150 explants error <10%; 200 explants error <6%
*Arabidopsis*
   For C24 ± 1000mg* *in vitro* plants (shoots + roots) (corresponds with -50 18-days old plants)
   Pregerminate seeds before sowing
   Grow for 18 days at 24°C
   (*cfr*. protocol *in vitro* growth Arabidopis)
      * for error <8%

Incubation media
*Brassica napus*
   25mM K-phosphate buffer pH5.8
   2% sucrose
   Tween20 (1 drop/25ml)
*Arabidopsis*
   10mM K-phosphate buffer pH5.8
   2% sucrose
   Tween20 (1 drop/25ml)
Before use, aerate (saturate with oxygen) medium well by stirring for at least a few hours

Assay
- Put explants in 100ml glass bottle (Schott, Germany) filled with incubation medium. Put the same weight of shoots in each bottle (± 700mg)
- Fill bottle to overflowing and close tightly (avoid large air bubbles)
- Fill also a bottle with incubation medium that does not contain explants (blanco)
- Incubate at 24°C at low light for: 3-4 hours (*Brassica napus*) 3 hours (*Arabidopsis*)
- Shake gently during incubation (to avoid oxygen depletion of medium around explants)
- Measure oxygen concentration (mg/l) of incubation media using an hand-held dissolved oxygen meter (Cyberscan DO 310; Eutech Instruments, Singapore)
- mg/l consumed oxygen = [oxygen] blanco - [oxygen] sample.

### Example 5. Analysis of transgenic plant lines comprising ParG gene expression reducing chimeric genes.

The chimeric genes of Example 2 were introduced into *Arabidopsis* an Nicotiana tabacum c.v. Petit Havana SR1 by *Agrobacterium* mediated transformation.

Transgenic seeds were germinated on a medium containing MS salts/2; B5 vitamins; 1,5% sucrose; pH5.8 and 0.7% Difco agar. Germinated seeds were subject to low light (photosynthetic photon flux of about 30 µmol m⁻¹ s⁻¹ for 14 to 18 days, after which the light intensity was increased about 6-fold (photosynthetic photon flux of about 190 µmol m⁻¹ s⁻¹). After 1 day, the NAD and NADH contents were determined using the enzymatic cycling method (Karp et al. (1983) Anal. Biochem. 128, pp 175-180). A portion of the seedlings were cultivated further under high light conditions for about 3 to about days, after which the damage was scored. Damage was visible as darkening of the young leaves and shoot tip, bleaching of older leaves and growth retardation. The results are summarized in Table 1 for Arabidopsis and in Table 2 for tobacco.

**Table 1. Analysis of Arabidopsis (Columbia). ±R indicates that some dark pigmentation was observed. ND: not determined**

| | High light tolerance | NAD+NADH content in 1 gram of tissue (10⁻³µM) | % TTC-reducing capacity vs control |
|---|---|---|---|
| Non-transgenic control | S | 17.3 | 100 |
| Transgenic line 9 | R | 28.2 | ND |
| Transgenic line 10 | R | 31.7 | ND |
| Transgenic line 11 | ±R | 26.5 | ND |
| Transgenic line 12 | S | 19.4 | ND |
| Transgenic line 26 | R | 33.2 | 55 |
| Transgenic line 27 | S | 21.3 | 100 |
| Transgenic line 28 | ±R | 26.5 | 75 |
| Transgenic line 29 | S | 17.7 | 102 |
| Transgenic line 30 | R | 28.3 | 66 |

**Table 2. Analysis of Nicotiana tabacum c.v. Petit Havana SR1. ±R indicates that some dark pigmentation was observed. R/S indicates tha the resistance phenotype was not very clear.**

| | High light tolerance | % TTC-reducing capacity vs control |
|---|---|---|
| Non-transgenic control | S | 100 |
| Transgenic line 1 | R/S | 88 |
| Transgenic line 2 | ±R | 79 |
| Transgenic line 3 | R | 53 |

There is a positive correlation between the resistance to high light stress in the transgenic plants and the NAD+NADH content of the cells. An inverse correlation can be seen between TTC reducing capacity and high light tolerance.

### Example 6. Construction of ParG gene expression reducing chimeric genes suited for use in cereal plants.

To reduce the expression of the PARG gene e.g. in cereals such as rice or corn (maize) and related plants, a chimeric gene is constructed which is capable expressing a dsRNA comprising both a sense and antisense region of nucleotide sequence from rice, that is capable of encoding a protein having high sequence identity with PARG protein encoding nucleotide sequences. The chimeric gene comprises the following DNA fragments:
- A promoter region from Cauliflower mosaic Virus (CaMV 35S);
- A DNA fragment comprising a sequence of at least 100 bp from ParG homologue from *Oryza sativa* (SEQ ID No 15) in direct orientation;
- A DNA fragment encoding intron 2 from the pdk gene from Flaveria;
- A DNA fragment comprising a sequence of at least 100 bp from ParG homologue from *Oryza sativa* (SEQ ID No 15) in inverted orientation;
- A fragment of the 3' untranslated end from the octopine synthetase gene from *Agrobacterium tumefaciens.*

This chimeric gene is introduced in a T-DNA vector, between the left and right border sequences from the T-DNA, together with a selectable marker gene providing resistance to e.g. the herbicide phosphinotricin.

To reduce the expression of the PARG gene e.g. in cereals such as rice or corn (maize) and related plants, a chimeric gene is constructed which is capable expressing a dsRNA comprising both a sense and antisense region of nucleotide sequence from rice, that is capable of encoding a protein having high sequence identity with PARG protein encoding nucleotide sequences. The chimeric gene comprises the following DNA fragments:
- A promoter region from Cauliflower mosaic Virus (CaMV 35S);
- A DNA fragment comprising a sequence of at least 100 bp from ParG homologue from Zea *mays* (SEQ ID No 23) in direct orientation;
- A DNA fragment encoding intron 2 from the pdk gene from Flaveria;
- A DNA fragment comprising a sequence of at least 100 bp from ParG homologue from Zea *mays* (SEQ ID No 23) in inverted orientation;
- A fragment of the 3' untranslated end from the octopine synthetase gene from *Agrobacterium tumefaciens.*

This chimeric gene is introduced in a T-DNA vector, between the left and right border sequences from the T-DNA, together with a selectable marker gene providing resistance to e.g. the herbicide phosphinotricin. The nucleotide sequence of two examples of such T-DNA vectors comprising two different chimeric gences as described in the previous paragraph is represented in SEQ ID Nos 24 and 25.

### Example 7. Analysis of transgenic plant lines comprising ParG gene expression reducing chimeric genes.

The chimeric genes of Example 6 are introduced into rice or corn respectively, by Agrobacterium mediated transformation.

The population of obtained transgenic lines is subjected to the following stress conditions, together with control plants:
- Increased heat for a period of days (greenhouse) or hours (in vitro)
- Drought for a period of days
- High light conditions for a period of days
- Nutrient depletion

Individual plant lines surviving well the above mentioned stress conditions, or at least one thereof, are selected.

The NAD content and ATP content for the above mentioned plants is determined under control and stress conditions.

## Claims

1. A method to produce a plant tolerant to stress conditions comprising the steps of
(a) providing plant cells with a chimeric gene to create transgenic plant cells, said chimeric gene comprising the following operably linked DNA fragments
(i) a plant-expressible promoter;
(ii) a DNA region, which when transcribed yields a ParG inhibitory RNA molecule;
(iii) a 3' end region involved in transcription termination and polyadenylation;
(b) regenerating a population of transgenic plant lines from said transgenic plant cell; and
(c) identifying a stress tolerant plant line within said population of transgenic plant lines.

2. The method according to claim 1, wherein said parG inhibitory RNA molecule comprises a nucleotide sequence of at least 20 consecutive nucleotides of the nucleotide sequence of the ParG gene present in said plant cell.

3. The method according to claim 1, wherein said parG inhibitory RNA molecule comprises a nucleotide sequence of at least 20 consecutive nucleotides of the complement of the nucleotide sequence of the ParG gene present in said plant cell.

4. The method according to claim 2 or 3, wherein said chimeric gene further comprises a DNA region encoding a self-splicing ribozyme between said DNA region coding for said parG inhibitory RNA molecule and said 3' end region.

5. The method according to claim 1, wherein said parG inhibitory RNA comprises a sense region comprising a nucleotide sequence of at least 20 consecutive nucleotides of the nucleotide sequence of the ParG gene present in said plant cell and an antisense region comprising a nucleotide sequence of at least 20 consecutive nucleotides of the complement of the nucleotide sequence of the ParG gene present in said plant cell, wherein said sense and antisense region are capable of forming a double stranded RNA region comprising said at least 20 consecutive nucleotides.

6. The method according to anyone of claims 1 to 5 wherein said stress conditions are selected from heat, drought, nutrient depletion, oxidative stress or high light conditions.

7. The method according to anyone of claims 1 to 6, comprising further crossing said transgenic plant line with another plant line to obtain stress tolerant progeny plants.

8. A DNA molecule comprising
(i) a plant-expressible promoter;
(ii) a DNA region, which when transcribed yields a ParG inhibitory RNA molecule;
(iii) a 3' end region involved in transcription termination and polyadenylation.

9. The DNA molecule according to claim 8, wherein said DNA region comprises a nucleotide sequence of at least 21 to 100 nucleotides of a nucleotide sequence encoding a protein comprising the amino acid sequence of SEQ ID No 1, 2 or 16 or at least 21 to 100 nucleotides of a nucleotide sequence of SEQ ID 3, 4, 15 or 23.

10. A plant cell comprising the DNA molecule of claim 8 or 9.

11. A plant consisting essentially of the plant cells of claim 10.

12. Seeds and propagating material of a plant according to claim 11, comprising the chimeric gene of claim 9 or 10.

13. A method to produce a plant tolerant to stress conditions comprising the steps of
(a) subjecting a plant cell line or a plant or plant line, to mutagenesis;
(b) identifying those plant cells or plants that have a mutation in an endogenous ParG gene resulting in a reduction of the PARG activity;
(c) subjecting the identified plant cells or plants to stress conditions;
(d) identifying plant cells or plants that tolerate said stress conditions better than control plants.

14. A method to produce a plant tolerant to stress conditions comprising the steps of
(a) selecting a plant cell line or a plant or plant line which is resistant to gallotannines;
(b) identifying those plant cells or plants that have a mutation in an endogenous ParG gene resulting in a reduction of the PARG activity;
(c) subjecting the identified plant cells or plants to stress conditions;
(d) identifying plant cells or plants that tolerate said stress conditions better than control plants.

## Patentansprüche

1. Verfahren zur Herstellung einer gegenüber Stressbedingungen toleranten Pflanze, umfassend die folgenden Schritte:
(a) Bereitstellen von Pflanzenzellen mit einem chimären Gen zur Erzeugung von transgenen Pflanzenzellen, wobei das chimäre Gen die folgenden operativ verknüpften DNA-Fragmente umfasst:
(i) einen in Pflanzen exprimierbaren Promoter;
(ii) eine DNA-Region, die, wenn sie transkribiert wird, ein ParG-hemmendes RNA-Molekül ergibt;
(iii) eine 3'-terminale Region, die an der Transkriptionstermination und der Polyadenylierung beteiligt ist;
(b) Regenerieren einer Population von transgenen Pflanzenlinien aus der transgenen Pflanzenzelle; und
(c) Identifizieren einer stresstoleranten Pflanzenlinie innerhalb der Population von transgenen Pflanzenlinien.

2. Verfahren nach Anspruch 1, wobei das ParG-hemmende RNA-Molekül eine Nukleotidsequenz mit mindestens 20 aufeinanderfolgenden Nukleotiden der Nukleotidsequenz des in der Pflanzenzelle vorhandenen ParG-Gens umfasst.

3. Verfahren nach Anspruch 1, wobei das ParG-hemmende RNA-Molekül eine Nukleotidsequenz mit mindestens 20 aufeinanderfolgenden Nukleotiden des Komplements der Nukleotidsequenz des in der Pflanzenzelle vorhandenen ParG-Gens umfasst.

4. Verfahren nach Anspruch 2 oder 3, wobei das chimäre Gen weiterhin eine DNA-Region, die für ein selbstspleißendes Ribozym codiert, zwischen der DNA-Region, die für das ParG-hemmende RNA-Molekül codiert, und der 3'-terminalen Region umfasst.

5. Verfahren nach Anspruch 1, wobei die ParG-hemmende RNA eine sense-Region umfassend eine Nukleotidsequenz mit mindestens 20 aufeinanderfolgenden Nukleotiden der Nukleotidsequenz des in der Pflanzenzelle vorhandenen ParG-Gens umfasst und eine antisense-Region umfassend eine Nukleotidsequenz mit mindestens 20 aufeinanderfolgenden Nukleotiden des Komplements der Nukleotidsequenz des in der Pflanzenzelle vorhandenen ParG-Gens umfasst, wobei die sense- und die antisense-Region fähig sind, eine Doppelstrang-RNA-Region umfassend mindestens 20 aufeinanderfolgende Nukleotide zu bilden.

6. Verfahren nach einem der Ansprüche 1 bis 5, wobei die Stressbedingungen aus der Reihe Hitze, Trockenheit, Nährstoffverarmung, Oxidationsstress oder Starklichtbedingungen ausgewählt sind.

7. Verfahren nach einem der Ansprüche 1 bis 6, das weiterhin umfasst, dass man die transgene Pflanzenlinie mit einer anderen Pflanzenlinie kreuzt, um stresstolerante Nachkommenschaftspflanzen zu erhalten.

8. DNA-Molekül, das Folgendes umfasst:
(i) einen in Pflanzen exprimierbaren Promoter;
(ii) eine DNA-Region, die, wenn sie transkribiert wird, ein ParG-hemmendes RNA-Molekül ergibt;

9. DNA-Molekül nach Anspruch 8, wobei die DNA-Region eine Nukleotidsequenz mit mindestens 21 bis 100 Nukleotiden einer Nukleotidsequenz, die für ein Protein umfassend die Aminosäuresequenz von SEQ ID No 1, 2 oder 16 codiert, oder mindestens 21 bis 100 Nukleotide einer Nukleotidsequenz von SEQ ID No 3, 4, 15 oder 23 umfasst.

10. Pflanzenzelle, die das DNA-Molekül nach Anspruch 8 oder 9 umfasst.

11. Pflanze, die im Wesentlichen aus den Pflanzenzellen nach Anspruch 10 besteht.

12. Samen und Vermehrungsmaterial einer Pflanze nach Anspruch 11, umfassend das chimäre Gen nach Anspruch 9 oder 10.

13. Verfahren zur Herstellung einer Pflanze, die gegenüber Stressbedingungen tolerant ist, umfassend die folgenden Schritte:
(a) Mutagenisieren einer Pflanzenzelllinie oder einer Pflanze oder einer Pflanzenlinie;
(b) Identifizieren derjenigen Pflanzenzellen oder Pflanzen, die eine Mutation in einem endogenen ParG-Gen aufweisen, die zu einer Reduktion der PARG-Aktivität führt;
(c) Aussetzen der identifizierten Pflanzenzellen oder Pflanzen an Stressbedingungen;
(d) Identifizieren der Pflanzenzellen oder Pflanzen, die die Stressbedingungen besser tolerieren als Kontrollpflanzen.

14. Verfahren zur Herstellung einer Pflanze, die gegenüber Stressbedingungen tolerant ist, umfassend die folgenden Schritte:
(a) Selektieren einer Pflanzenzelllinie oder einer Pflanze oder einer Pflanzenlinie, die gegenüber Gallotanninen resistent ist;
(b) Identifizieren derjenigen Pflanzenzellen oder Pflanzen, die eine Mutation in einem endogenen ParG-Gen aufweisen, die zu einer Reduktion der PARG-Aktivität führt;
(c) Aussetzen der identifizierten Pflanzenzellen oder Pflanzen an Stressbedingungen;
(d) Identifizieren der Pflanzenzellen oder Pflanzen, die die Stressbedingungen besser tolerieren als Kontrollpflanzen.

## Revendications

1. Procédé de production d'une plante tolérante à des conditions de stress, comprenant les étapes de
(a) mise à disposition de cellules végétales comportant un gène chimère pour créer des cellules de plantes transgéniques, ledit gène chimère comprenant les fragments d'ADN liés d'une manière opérationnelle suivants :
(i) un promoteur exprimable dans des plantes ;
(ii) une région d'ADN qui, après transcription, donne une molécule d'ARN inhibitrice de ParG ;
(iii)une région 3'-terminale impliquée dans la terminaison de transcription et dans la polyadénylation ;
(b) régénération d'une population de lignées de plantes transgéniques à partir de ladite cellule de plantes transgéniques ; et
(c) identification d'une lignée de plantes tolérantes au stress, à l'intérieur de ladite population de lignées de plantes transgéniques.

2. Procédé selon la revendication 1, dans lequel ladite molécule d'ARN inhibitrice de ParG comprend une séquence nucléotidique d'au moins 20 nucléotides consécutifs de la séquence nucléotidique du gène ParG présent dans ladite cellule végétale.

3. Procédé selon la revendication 1, dans lequel ladite molécule d'ARN inhibitrice de ParG comprend une séquence nucléotidique d'au moins 20 nucléotides consécutifs du complément de la séquence nucléotidique du gène ParG présent dans ladite cellule végétale. séquence nucléotidique d'au moins 20 nucléotides consécutifs du complément de la séquence nucléotidique du gène ParG présent dans ladite cellule végétale.

4. Procédé selon la revendication 2 ou 3, dans lequel ledit gène chimère comprend en outre une région d'ADN codant pour un ribozyme à auto-épissage entre ladite région d'ADN codant pour ladite molécule d'ARN inhibitrice de ParG et ladite région 3'-terminale.

5. Procédé selon la revendication 1, dans lequel ledit ARN inhibiteur de ParG comprend une région sens comprenant une séquence nucléotidique d'au moins 20 nucléotides consécutifs de la séquence nucléotidique du gène ParG présent dans ladite cellule végétale et une région antisens comprenant une séquence nucléotidique d'au moins 20 nucléotides consécutifs du complément de la séquence nucléotidique du gène ParG présent dans ladite cellule végétale, ladite région sens et ladite région antisens étant capables de former une région d'ARN double brin comprenant lesdits au moins 20 nucléotides consécutifs.

6. Procédé selon l'une quelconque des revendications 1 à 5, dans lequel lesdites conditions de stress sont choisies parmi la chaleur, la sécheresse, l'épuisement en nutriments, le stress oxydatif ou les conditions d'une forte luminosité.

7. Procédé selon l'une quelconque des revendications 1 à 6, qui comprend en outre le croisement de ladite lignée de plantes transgéniques avec une autre lignée végétale pour obtenir des plantes de descendance tolérantes au stress.

8. Molécule d'ADN, comprenant :
(i) un promoteur exprimable dans des plantes ;
(ii) une région d'ADN qui, après transcription, donne une molécule d'ARN inhibitrice de ParG ;

9. Molécule d'ADN selon la revendication 8, dans laquelle ladite région d'ADN comprend une séquence nucléotidique d'au moins 21 à 100 nucléotides d'une séquence nucléotidique codant pour une protéine comprenant la séquence d'acides aminés de SEQ ID N°1, 2 ou 16, ou au moins 21 à 100 nucléotides d'une séquence nucléotidique de SEQ ID N°3, 4, 15 ou 23.

10. Cellule végétale comprenant la molécule d'ADN de la revendication 8 ou 9.

11. Plante consistant essentiellement en des cellules végétales de la revendication 10.

12. Graines et matériel de reproduction d'une plante selon la revendication 11, comprenant le gène chimère de la revendication 9 ou 10.

13. Procédé de production d'une plante tolérante à des conditions de stress, comprenant les étapes de
(a) soumission d'une lignée de cellules végétales ou d'une plante ou d'une lignée végétale à une mutagenèse ;
(b) identification des cellules végétales ou des plantes qui possèdent une mutation dans un gène ParG endogène, conduisant à une réduction de l'activité de ParG ;
(c) soumission des cellules végétales ou des plantes identifiées à des conditions de stress;
(d) identification des cellules végétales ou des plantes qui tolèrent lesdites conditions de stress mieux que des plantes témoins.

14. Procédé de production d'une plante tolérante à des conditions de stress, comprenant les étapes de
(a) sélection d'une lignée de cellules végétales ou d'une plante ou d'une lignée végétale qui est résistante au gallotannines ;
(b) identification des cellules végétales ou des plantes qui possèdent une mutation dans un gène ParG endogène, conduisant à une réduction de l'activité de ParG ;
(c) soumission des cellules végétales ou des plantes identifiées à des conditions de stress ;
(d) identification des cellules végétales ou des plantes qui tolèrent lesdites conditions de stress mieux que des plantes témoins.
